# EUROPEAN PATENT SPECIFICATION

(11) **EP 4 419 535 B1**
(45) Date of publication and mention of the grant of the patent: **14.01.2026**
(21) Application number: 22803247.0
(22) Date of filing: 18.10.2022
(51) Int. Cl.: C07H 15/256, A23L 2/60, A23L 27/30

(54) **RUBUSOSIDE GLUCOSIDES**
RUBUSOSIDGLUCOSIDE
GLUCOSIDES RUBUSOSIDES

(30) Priority: 19.10.2021 WO PCT/EP2021/078871
(43) Date of publication of application: 28.08.2024
(73) Proprietor: Symrise AG, 37603 Holzminden Niedersachsen (DE)
(72) Inventor: WELLMANN, Johannes, 37603 Holzminden (DE); LEY, Jakob Peter, 37603 Holzminden (DE); HILLEBRAND, Silke, 38159 Vechelde (DE); WILMS, Jonas, 86738 Deiningen (DE); SOMERS, Tom, 37603 Holzminden (DE); RIESS, Thomas, 37603 Holzminden (DE); HARTMANN, Beate, 33034 Brakel (DE)
(74) Representative: Eisenführ Speiser
(86) International application number: PCT/EP2022/078949
(87) International publication number: WO 2023/066922

(56) References cited:
- WO-A1-2015/189346
- WO-A2-2013/019050
- WO-A2-2014/172055

## Description

The invention relates to a novel compound, a mixture comprising the compound, a method for producing such a compound, a compound obtained or obtainable by such a method, a mixture comprising one or more such compound(s), a flavour composition comprising such a compound or mixture, a composition serving for food or pleasure or pharmaceutical composition comprising one or more of such compounds or such a mixture, as well as to the use of such a compound or of such a mixture for imparting, modifying and/or enhancing a sweet taste impression and a method for imparting, modifying and/or enhancing a sweet taste impression.

Foodstuff as well as drinks and further consumable products with a high content of sugar (particularly sucrose, lactose, glucose, fructose or mixtures thereof) are particularly preferred by consumers as they provide a sweet taste impression.

However, at the same time, it is known that a high content of carbohydrates, which can be metabolized quickly, lead to an increase in the blood glucose level, the formation of fat deposits and finally result in health conditions such as excess weight or obesity, insulin resistance, adult-onset diabetes and their secondary diseases.

Furthermore, several of such carbohydrates may negatively affect dental health, as they can be metabolized by several bacteria, which are present in the oral cavity. The metabolites of these carbohydrates may in turn degrade the tooth enamel and finally lead to caries.

Since long time, it is thus an aim to reduce the sugar content of foodstuff and drinks as far as possible, without significantly affecting the perceived sweetness.

One measure is to replace the carbohydrates by sweeteners. Sweeteners are chemical compounds, which have no or only a small caloric value and, at the same time, provide a strong sweet taste impression. An overview of such sweeteners may, for example, be found in the Journal of the American Dietetic Association 2004, 104 (2), 255-275).

With their high sweetening power, these sweeteners may be added to foodstuff in low amounts. In this way, it is possible to replace large amounts of carbohydrates with low amounts of sweeteners.

Many of such sweeteners are compounds that can be found in nature and that are frequently extracted from several plants. Rubusoside is a sweetener, which is found in *Rubus suavissimus.* Rubusoside provides a sweet taste impression, which is reported to be 200 times as strong as sucrose and is thus particularly suitable as a sweetener, as described above.

In addition to Rubusoside, or instead of Rubusoside, glycosides of Rubusoside are used as sweeteners. Such glycosides are typically also obtained from the leaves of the plant *Rubus suavissimus* or can be obtained by glycosylation of Rubusoside.

Products containing distinct Rubusoside glycosides as sweeteners are known from the state of the art. Attempts to increase the content of Rubusoside by concentration processes are also known. For example, mixtures of Rubusoside and stevioside and their use as sweeteners are described in the two patent applications EP 2641479 and WO 2012/177727.

Previously, individual products of an alpha-glucosylation of Rubusoside were isolated and evaluated for their taste (Ohtani, K.; Aikawa, Y.; Ishikawa, H.; Kasai, R.; Kitahata, S.; Mizutani, K.; Doi, S.; Nakaura, M.; Tanaka, O., Further study on the 1,4-alpha-transglucosylation of Rubusoside, a sweet steviol-bisglucoside from Rubus suavissimus. Agric. Biol. Chem. 1991, 55, (2), 449-453), however, the purification until a single substance is obtained is extremely complex: Therefore, such processes are commercially not or only hardly reasonable, which strongly restricts their applicability.

Furthermore, WO 2015/189346 describes the transglycosylation of Rubusoside and their use as taste balancing substances.

Further Rubusoside derivatives are disclosed in WO2014/172055 A2 and WO 2013/019050 A2.

The glucosides of Rubusoside, which are mainly used, are the α-1,4-glucosides. Their sensory effect is described in, for example, Ohtani, K., et al. (1991). "Further study on the 1,4-alpha-transglucosylation of Rubusoside, a sweet steviol-bisglucoside from Rubus suavissimus" Agric. Biol. Chem. 55(2): 449-453.

1,2-β- and 1,3-β- as well as 1,6-α-mono-glucosylated Rubusosides are described in Zhao, L., et al. (2020). "Enzymatic Monoglucosylation of Rubusoside and the Structure-Sweetness/Taste Relationship of Monoglucosyl Derivatives." Journal of Agricultural and Food Chemistry 68(32): 8702-8709. It is disclosed, particularly in table 1, that different Rubusoside glucosides provide different sweetness and, additionally, a different quality of taste, depending on the glucosidic bond and the glucosylation site (i.e. either at C₁₃ or at C₁₉). Remarkably, a 1,3-β-glucosylation provided a strongly lower sweetness and quality of taste, independent of the glucosylation site. Consequently, not all of the Rubusoside glucosides are suitable for a use as (potent) sweetener for replacing sugar from foodstuff. Particularly, 1,3-β-glucosylation does not seem to provide suitable Rubusoside glucosides.

Furthermore, one major drawback of α-1,4-glucosides of Rubusoside is that these are degraded by the enzyme amylase in the oral cavity. Amylases are present in the saliva of humans, where they begins the chemical process of digestion. Particularly, amylases catalyse the hydrolysis of sugar polymers, such as starch, into smaller polymers or even into monomers. The α-amylase ptyalin is the amylase present in human saliva and thus also called salivary amylase. Ptyalin particularly hydrolyses α-1,4-glycosidic bonds and thus degrades the α-1,4-glucosides of Rubusoside in the oral cavity.

It was thus one object of the present invention to provide novel glucosides of Rubusoside, which provide an advantageous sweetness and quality of taste. Preferably, such glucosides shall be more stable against degradation in the oral cavity than α-1,4-glucosides of Rubusoside.

The primary object of the present invention is solved by a compound according to the following formula

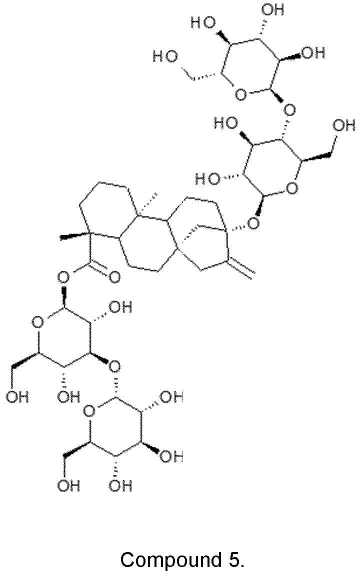

It is advantageous that the compound according to the invention provides a particularly beneficial sweet taste impression and a particularly beneficial taste profile, as shown in Example 4.

Furthermore, it is advantageous that the compound according to the invention is more stable with regard to a saliva-based or, respectively amylase-based, metabolism.

In case it is referred to a chemical structure and to a compound name or description of the compound and the structure and name or description do not correspond to each other, it is the chemical structure, which prevails.

Furthermore, the invention relates to a mixture comprising the compound according to the invention.

It is preferred that the mixture according to the invention further comprises the compound according to the following formula

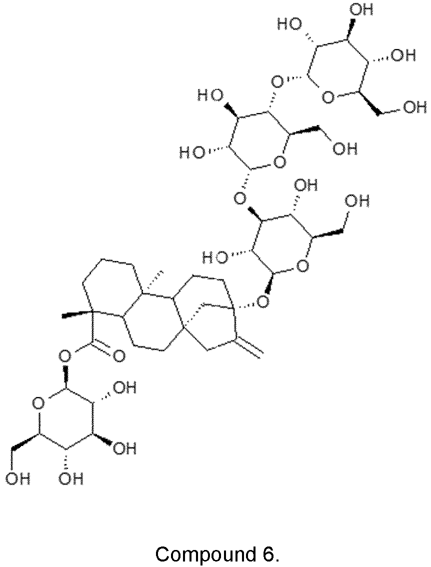

It is preferred that the mixture according to the invention comprises
a) 40 to 90 wt.-% Compound 5 and
b) 10 to 60 wt.-% Compound 6,
based on the total weight of the mixture.

Preferably, in the mixture according to the invention, the weight ratio of Compound 5 and Compound 6 is in a range of from 10:1 to 1:5, preferably in a range of from 7.5:1 to 1:4, preferably in a range of from 5:1 to 1:3, preferably in a range of from 3:1 to 1:2, preferably in a range of from 2.5:1 to 1:1.5.

The invention further relates to a method for producing a compound according to the invention, comprising the following steps:
(i) Providing Rubusoside and providing a glucosyl donor, mixing the provided Rubusoside and the glucosyl donor,
(ii) dissolving the mixture of step (i) in a solvent, preferably water,
(iii) adding a glucanotransferase and reacting the dissolved mixture with the glucanotransferase to obtain glucosylated Rubusosides,
(iv) heating the mixture for inactivating the glucanotransferase,
(v) enriching the glucosylated Rubusosides in the mixture, preferably by column chromatography,
(vi) optionally: separating the enriched glucosylated Rubusosides of the mixture, preferably by liquid chromatography.

The term "Rubusoside" as described in step (i) of the method according to the invention describes the following compound 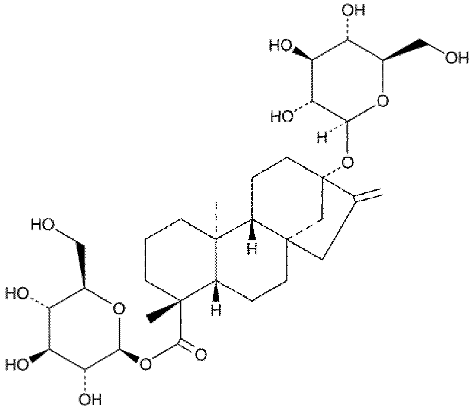 as well as any of its stereoisomers. Preferably, the term "Rubusoside" as described in step (i) of the method according to the invention additionally describes any glycoside of the above compound and its stereoisomers.

The term "glucosyl donor" as described in step (i) of the method according to the invention describes any compound, which provides a glucose monomer in a chemical reaction, wherein the glucose monomer will be connected to Rubusoside via a new glycosidic bond in the same or in a subsequent chemical reaction. Preferably, the glucose monomer of the glucosyl donor is connected to the glucosyl donor, before the chemical reaction, via an α-1,4-glycosidic bond. Preferably, the glucosyl donor is an α-1,4-glucan. Preferably, the glucosyl donor is selected from the group consisting of starch, dextrins, maltodextrins, maltooligosaccharides, maltotetrose, maltotriose, maltobiose, cyclodextrins and mixtures thereof. Particularly the cyclodextrins, as described in the group above, are alpha, beta or gamma cyclodextrins or mixtures thereof.

The term "glucosylated Rubusosides", as used herein, describes Rubusoside, as described herein, which further includes at least one glucose monomer, as described herein. Thus, in case a glycoside of Rubusoside is used as "Rubusoside", as described above, the term "glucosylated Rubusoside" describes this glycoside of Rubusoside, which contains at least one additional glucose monomer.

The term "enriching the glucosylated Rubusosides in the mixture", as used herein, describes any suitable process to increase the concentration of the glucosylated Rubusosides in the mixture, such as removing further components or solvents from the mixture or performing a column chromatography.

The term "separating the enriched glucosylated Rubusosides", as used herein, describes any suitable process for separation according to any desired parameter. Preferably, the glucosylated Rubusosides are separated according to their degree of glycosylation or glucosylation. Additionally or alternatively, the glucosylated Rubusosides are separated into specific isomers. Preferably, the separation is achieved by two or more separation steps, particularly preferably, wherein the separation parameter is different from the previous and the subsequent step, if applicable, especially preferably, wherein the separation parameter is different in each step.

It is preferred that the method according to the invention comprises step (vi) and step (vi) comprises a first liquid chromatography, in which the glucosylated Rubusosides are separated into different fractions according to their degree of glucosylation, and a second liquid chromatography, in which the obtained fractions are further separated into the specific isomers of the glucosylated Rubusosides. Thus, the glucosylated Rubusosides are first separated by the parameter "degree of glucosylation" and afterwards by the parameter "isomers". Preferably, for separating the glucosylated Rubusosides by the parameter "degree of glucosylation", the liquid chromatography may be performed with a HILIC (hydrophilic interaction liquid chromatography) phase. Additionally or alternatively, it is preferred that for separating the glucosylated Rubusosides by the parameter "isomers", the liquid chromatography may be performed with a RP (reversed phase) phase.

Preferably, the term "separating" as used herein, preferably as used in the context of the parameter "degree of glucosylation" or preferably as used in the context of the parameter "isomers", is to be understood as including a separation but does not require a complete separation of each of the different compounds from each other. I.e. the term "separating" only requires that at least two subgroups are obtained, which differ from each other by means of the respective parameter of separation, preferably wherein the parameter of separation is determined as average over the respective subgroup.

The invention further relates to Compound 5 obtained or obtainable by a method according to the invention.

Further, the invention relates to a mixture comprising Compound 5 obtained or obtainable by a method according to the invention. Preferably, this mixture further comprises Compound 6 as described herein.

Particularly preferably, this mixture comprises
a) 40 to 90 wt.-% of Compound 5 obtained or obtainable by a method according to the invention and
b) 10 to 60 wt.-% Compound 6,
based on the total weight of the mixture.

Preferably, in this mixture, the weight ratio of Compound 5 obtained or obtainable by a method according to the invention and Compound 6 is in a range of from 10:1 to 1:5, preferably in a range of from 7.5:1 to 1:4, preferably in a range of from 5:1 to 1:3, preferably in a range of from 3:1 to 1:2, preferably in a range of from 2.5:1 to 1:1.5.

Moreover, the invention relates to a mixture comprising Compound 5 and optionally Compound 6, wherein the mixture is obtained or obtainable by a method according to the invention.

Particularly preferably, the mixture obtained or obtainable by a method according to the invention comprises
a) 40 to 90 wt.-% of Compound 5 and
b) 10 to 60 wt.-% Compound 6,

based on the total weight of the mixture,
and preferably wherein Compound 5 and Compound 6 are obtained or obtainable by a method according to the invention.

Preferably, in the mixture obtained or obtainable by a method according to the invention, the weight ratio of Compound 5 and Compound 6 is in a range of from 10:1 to 1:5, preferably in a range of from 7.5:1 to 1:4, preferably in a range of from 5:1 to 1:3, preferably in a range of from 3:1 to 1:2, preferably in a range of from 2.5:1 to 1:1.5, preferably wherein Compound 5 and Compound 6 are obtained or obtainable by a method according to the invention.

Furthermore, the invention relates to a flavour composition comprising Compound 5, preferably obtained or obtainable by a method according to the invention.

Furthermore, the invention relates to a flavour composition comprising a mixture according to the invention.

Preferably, in the flavour composition according to the invention, the total weight of Compound 5, preferably obtained or obtainable by a method according to the invention, is at least 0.05 wt.-%, preferably at least 0.1 wt.-%, particularly preferably at least 0.25 wt.-%, based on the total weight of the flavour composition.

Preferably, in the flavour composition according to the invention, the total weight of Compound 5, preferably obtained or obtainable by a method according to the invention, and Compound 6 is at least 0.05 wt.-%, preferably at least 0.1 wt.-%, particularly preferably at least 0.25 wt.-%, based on the total weight of the flavour composition.

The term "the total weight of Compound 5 and Compound 6", as used herein, refers to the summed amount of Compound 5 and Compound 6.

Preferably, in the flavour composition according to the invention, the total weight of Compound 6, preferably obtained or obtainable by a method according to the invention, is at least 0.05 wt.-%, preferably at least 0.1 wt.-%, particularly preferably at least 0.25 wt.-%, based on the total weight of the flavour composition.

The invention further relates to a composition serving for food or pleasure or a pharmaceutical composition comprising Compound 5, preferably obtained or obtainable by a method according to the invention, or a mixture according to the invention or a flavour composition according to the invention.

Preferably, the flavour composition according to the invention, the composition according to the invention serving for food or pleasure or the pharmaceutical composition according to the invention may be present in a form selected from the group consisting of tables (non-coated as well as coated tablets, single or multiple layered tablets), capsules, lozenges, granules, pellets, solid substance mixtures, dispersions in liquid phases, emulsions, powders, solutions, juices, pastes or other swallowable or chewable preparations.

Preferably, the composition according to the invention serving for food or pleasure may be selected from the group consisting of
baked goods, for example, bread, dry biscuits, cakes, other baked products, confectionery (for example, chocolate, chocolate bar products, other bar products, fruit gum, hard and soft caramels, chewing gum), alcoholic or nonalcoholic beverages (for example, coffee, tea, iced tea, wine, wine-containing beverages, beer, beer-containing beverages, liqueurs, brandies, (carbonated) fruit-containing beverages, (carbonated) isotonic beverages, (carbonated) soft beverages, nectars, spritzers, fruit and vegetable juices, fruit or vegetable juice formulations, instant beverages (for example, instant cocoa beverages, instant tea beverages, instant coffee beverages, instant fruit beverages), meat products (for example, ham, fresh sausage or uncooked sausage formulations, seasoned or marinated fresh or salted meat products), eggs or egg products (dried egg, egg white, egg yolk), cereal products (for example, breakfast cereals, muesli bars, precooked ready-made rice products), dairy products (for example, milk beverages, buttermilk beverages, milk ice, yoghurt, kefir, fresh cheese, soft cheese, hard cheese, dried milk powder, whey, whey beverages, butter, buttermilk, products containing partly or completely hydrolysed milk protein), products made of soy protein or other soybean fractions (for example, soy milk and products produced thereof, fruit beverages with soy protein, soy lecithin-containing formulations, fermented products such as tofu or tempe or products produced thereof), products made of other plant-based protein sources, for example, oat protein beverages, fruit preparations (for example, preserves, fruit ice-cream, fruit sauces, fruit fillings), vegetable preparations (for example, ketchup, sauces, dried vegetables, frozen vegetables, precooked vegetables, vegetables preserved in vinegar), snacks (for example, baked or fried potato chips (crisps) or potato dough products, extrudates based on maize or peanuts), fat- and oil-based products or emulsions thereof (for example, mayonnaise, remoulade, dressings), other ready-made dishes and soups (for example dried soups, instant soups, precooked soups), spices or spice formulations and particularly seasonings, which are used, for example, in the snacks sector.

Particularly preferred herein are sweets, dairy products and very particularly preferred are non-alcoholic beverages where sweetened beverages are particularly preferred.

Furthermore, the flavour composition according to the invention, the composition according to the invention serving for food or pleasure or the pharmaceutical composition according to the invention may contain one or more further additives and adjuvants.

Particularly the flavour composition according to the invention, the composition according to the invention serving for food or pleasure or the pharmaceutical composition according to the invention may contain one or more further sweeteners and/or one or more further aroma substances.

Preferably, the, one or more or all further sweeteners may be selected from the group consisting of carbohydrates and specifically sugars such as, for example, sucrose/saccharose, trehalose, lactose, maltose, melizitose, raffinose, palatinose, lactulose, D-fructose, D-allulose, D-glucose, D-galactose, L-rhamnose, D-sorbose, D-mannose, D-tagatose, D-arabinose, L-arabinose, D-xylose, D-ribose, D-glyceraldehyde or maltodextrin; synthetic, i.e. usually enzymatically produced, starch or sugar hydrolysates (invert sugar, fructose syrup); fruit concentrates (e.g., on the basis of apples or pears); sugar alcohols (e.g., erythritol, threitol, arabitol, ribotol, xylitol, sorbitol, mannitol, dulcitol, lactitol); proteins (e.g., miraculin, monellin, thaumatin, curculin, brazzein); sweeteners (e.g., magap, sodium cyclamate, acesulfam K, neohesperidin dihydrochalcone, saccharine sodium salt, aspartame, superaspartame, neotame, alitame, sucralose, stevioside, rebaudioside, lugduname, carrelame, sucrononate, sucrooctate, monatin, phenylodulcin); sweet-tasting amino acids (e.g., glycine, D-leucine, D-threonine, D-asparagine, D-phenylalanine, D-tryptophan, L-proline); further sweet-tasting low-molecular substances such as, e.g., hernandulcin, dihydrochalcon glycoside, particularly, neohesperidin dihydrochalcone, and naringin chalcone, glycyrrhizin, glycerrhetinic acid, their derivatives and salts, extracts of liquorice (Glycyrrhizza glabra ssp.), Lippia dulcis extracts, Momordica ssp. Extracts; individual substances such as, for example, Momordica grosvenori [Luo Han Guo] and the mogrosides obtained thereof, Hydrangea dulcis or extracts, phyllodulcin; balansins of Mycetia balansae.

Also preferably, the, one or more or all further aroma substances ay be selected from the group consisting of acetophenone, allyl caproate, alpha-ionone, beta-ionone, aniseed aldehyde, anisyl acetate, anisyl formate, benzaldehyde, benzothiazole, benzyl acetate, benzyl alcohol, benzyl benzoate, beta-ionone, butyl butyrate, butyl capronate, butylidene phthalide, carvone, camphene, caryophyllene, cineol, cinnamyl acetate, citral, citronellol, citronellal, citronellyl acetate, cyclohexyl acetate, cymol, damascone, decalactone, dihydrocoumarin, dimethyl anthranilate, dimethyl anthranilate, dodecalactone, ethoxy ethyl acetate, ethylbutyric acid, ethyl butyrate, ethyl caprinate, ethyl capronate, ethyl crotonate, ethyl furaneol, ethylguaiakol, ethyl isobutyrate, ethyl isovalerianate, ethyl lactate, ethylmethyl butyrate, ethyl propionate, eucalyptol, eugenol, eugenyl acetate, ethyl heptylate, 4-(p-hydroxyphenyl)-2-butanone, gamma-decalactone, geraniol, geranyl acetate, geranyl acetate, grapefruit aldehyde, methyl dihydrojasmonate (e.g., 5 Hedion^{®}), heliotropin, 2-heptanone, 3-heptanone, 4-heptanone, trans-2-heptenal, cis-4-heptenal, trans-2-hexenal, cis-3-hexenol, trans-2-hexenoic acid, trans-3-hexenoic acid, cis-2-hexenyl acetate, cis-3-hexenyl acetate, cis-3-hexenyl capronate, trans-2-hexenyl capronate, cis-3-hexenyl formiate, cis-2-hexyl acetate, cis-3-hexyl acetate, trans-2-hexyl acetate, cis-3-hexyl formiate, para-hydroxybenzyl acetone, isoamyl alcohol, isoamyl isovalerianate, isobutyl butyrate, isobutyl aldehyde, isoeugenol methyl ether, isopropyl methyl thiazole, lauric acid, levulinic acid, linalool, linalool oxide, linalyl acetate, menthol, menthofuran, methyl anthranilate, methyl butanol, methyl butyric acid, 2-methyl butyl acetate, methyl capronate, methyl cinnamate, 5-methyl furfural, 3,2,2-methyl cyclopentenolone, 6,5,2-methyl heptenone, methyl dihydrojasmonate, methyljasmonate, 2-methyl methylbutyrate, 2-methyl-2-pentanoic acid, methyl thiobutyrate, 3,1-methyl thiohexanol, 3-methyl thiohexyl acetate, nerol, neryl acetate, trans,trans-2,4-nonadienal, 2,4-nonadienol, 2,6-nonadienol, 2,4-nonadienol, nootkatone, delta octalactone, gamma octalactone, 2-octanol, 3-octanol, 1,3-octenol, 1-octyl acetate, 3-octyl acetate, palmitic acid, paralde-hyde, phellandrene, pentandione, phenylethyl acetate, phenylethyl alcohol, phenylethyl alcohol, phenylethyl isovalerianate, piperonal, propionaldehyde, propyl butyrate, pulegon, pulegol, sinensal, sulfurol, terpinene, terpineol, terpinolene, 8,3-thiomenthanone, 4,4,2-thiomethyl pentanone, thymol, delta-undecalactone, gamma-undecalactone, valencene, valeric acid, vanilline, acetoine, ethyl vanilline, ethyl vanilline isobutyrate (= 3-ethoxy-4-isobutyryloxybenzaldehyde), 2,5-dimethyl-4-hydroxy-3(2H)-furanone and derivatives (here, preferably homofuraneol (= 2-ethyl-4-hydroxy-5-methyl-3(2H)-furanone), homofuronol (= 2-ethyl-5-methyl-4-hydroxy-3(2H)-furanone and 5-ethyl-2-methyl-4-hydroxy-3(2H)-furanone), maltol and maltol derivatives (here, preferably ethyl maltol), coumarine and coumarine derivatives, gamma-lactones (here, preferably gamma-undecalactone, gamma-nonalactone, gamma-decalactone), delta-lactones (here, preferably 4-methyldeltadecalactone, massoilactone, delta-decalactone, tubero lactone), methyl sorbate, divanilline, 4-hydroxy-2(or 5)-ethyl-5(or 2)-methyl-3(2H)furanone, 2-hydroxy-3-methyl-2-cyclopentenon, 3-hydroxy-4,5-dimethyl-2(5H)-furanone, isoamyl acetate, ethyl butyrate, butyl butyrate, isoamyl butyrate, methyl-3-ethyl butyrate, n-hexanoic acid allyl ester, n-hexanoic acid-n-butyl ester, n-ethyl octanoate, ethyl-3-methyl-3-phenylglycidate, ethyl-2-trans-4-cis-decadienoate, 4-(p-hydroxyphenyl)-2-butanone, 1,1-dimethoxy-2,2,5-trimethyl-4-hexane, 2,6-dimethyl-5-hepten-1-al and phenylacetaldehyde, 2-methyl-3-(methylthio)furane, 2-methyl-3-furanthiol, bis(2-methyl-3-furyl)disulfide, furfuryl mercaptane, methional, 2-acetyl-2-thiazoline, 3-mercapto-2-pentanone, 2,5-dimethyl-3-furanthiol, 2,4,5-trimethylthiazol, 2-acetylthiazol, 2,4-dimethyl-5-ethylthiazol, 2-acetyl-1-pyrroline, 2-methyl-3-ethylpyrazine, 2-ethyl-3,5-dimethylpyrazine, 2-ethyl-3,6-dimethylpyrazine, 2,3-diethyl-5-methylpyrazine, 3-isopropyl-2-methoxypyrazine, 3-Isobutyl-2-methoxypyrazine, 2-acetylpyrazine, 2-pentylpyridine, (E,E)-2,4-decadienal, (E,E)-2,4-nonadienal, (E)-2-octenal, (E)-2-nonenal, 2-undecenal, 12-methyltridecanal, 1-Penten-3-one, 4-hydroxy-2,5-dimethyl-3(2H)-furanone, guaiakol, 3-hydroxy-4,5-dimethyl-2(5H)-furanone, 3-hydroxy-4-methyl-5-ethyl-2(5H)-furanone, cinnamon aldehyde, cinnamon alcohol, methyl salicylate, isopulegol; lactisoles, lactisol esters such as described in EP 2,292,224, sodium salts (e.g., sodium chloride, sodium lactate, sodium nitrate, sodium acetate, sodium gluconate), hydroxyflavanones, here, preferably eriodictyol, sterubin (eriodictyol-7-methyl ether), homoeriodictyol, and their sodium, potassium, calcium, magnesium or zinc salts (particularly those as described in EP 1258200 A2) divanillins (particularly those as described in WO 2004/078302) and 4-hydroxydihydrochalcones (preferably as described in US 2008/0227867 A1), here, particularly phloretin and davidigenin, umami compounds as described in WO 2008/046895 A1 and EP 1989944 A1, umami compounds as described in EP 2064959 A1, EP 2,529,632 B1 or EP 2135516 A1, here particularly preferably rubemamine and rubescenamine, vanillyl lignans, particularly preferably lariciresinol or matairesinol as described in WO 2012/146584, enterodiol as described in DE 10 2012 214 560, alkamides, particularly pellitorines as described in EP 2,058,297, EP 1,977,655 B1 and EP 2,008,530 B1, and N-decadienoyl amino acids as described in EP 2,597,082 and their mixtures, as well as stereoisomers, enantiomers, positional isomers, diastereomers, cis/trans-isomers and epimers of these substances.

It is further preferred that the pharmaceutical composition according to the invention can be present in form of tables (non-coated as well as coated tablets, single or multiple layered tablets), capsules, lozenges, granules, pellets, solid substance mixtures, dispersions in liquid phases, emulsions, powders, solutions, juices, pastes or other swallowable or chewable preparations.

Furthermore, the invention relates to the use of Compound 5, preferably obtainable or obtained by a method according to the invention, or of a mixture according to the invention or of a flavour composition according to the invention, for imparting, modifying and/or enhancing a sweet taste impression.

Moreover, the invention relates to a method for imparting, modifying and/or enhancing a sweet taste impression, comprising the following steps:
(i) Providing Compound 5, preferably obtained or obtainable by a method according to the invention, or a mixture according to the invention or a flavour composition according to the invention,
(ii) mixing the compound or mixture or flavour composition provided in step (i) with a substance or product, of which a sweet taste impression shall be imparted, modified and/or enhanced.

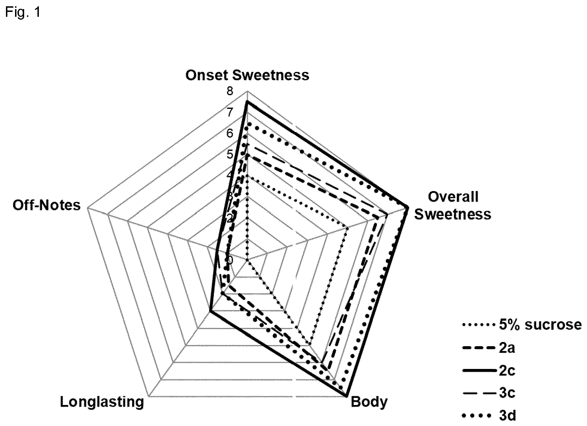

Fig. 1 depicts the results of the sensory evaluation (as in Example 4.2) of the compound according to the invention, with 5 % sucrose solution (control), 2a (5 % sucrose and 80 ppm of compound 3, control), 2b (5 % sucrose and 80 ppm of a mixture of compounds 5 and 6, according to the invention), 3a (5 % sucrose and 80 ppm of a mixture of compounds 9 and 10, control) and 3b (5 % sucrose and 80 ppm of compound 11, control).

Further aspects and advantages of the invention result from the subsequent description of preferred examples.

### Examples

### Example 1: Preparation of a mixture of glycosylated Rubusosides

100 g of each, Maltodextin (DE8 from maize; Symrise) and Rubusoside (approx.. 95 %; Hunan Nutramax, Changsha City, Hunan Province, China, 410016), were provided. 500 mL town water were added and it was stirred until all components were completely dissolved. After addition of an enzyme solution (Gluconotransferase Toryzyme 3.0 L; Novozymes), the sample was shaken at 50 % and 160 rpm for 6 hours to react the dissolved mixture with the glucanotransferase for obtaining glycosylated Rubusosides. Subsequently, the mixture was heated to 80 °C for 10 min for inactivating the enzyme. After cooling down, the mixture was freeze-dried. The mixture contains a residual amount of non-glucosylated Rubusoside (as initially provided) of approximately 11 % (i.e. approximately 77 % have been glucosylated).

### Example 2: Isolation of single compounds

The glycosylated Rubusosides were enriched via a hydrophobic resin (XAD-1180, Sigma Aldrich): 50 g of the mixture obtained in Example 1 were dissolved in 350 mL water and applied on a column of 20 mL hydrophobic resin. The column was previously and subsequently equilibrated with water. The loaded column was washed with 400 mL water. Subsequently, the glycosylated Rubusosides were eluted with 70 % ethanol. The eluate is vaporized by vacuum vaporization and lyophilisation until being dry. The amount of Rubusoside in the enriched glycosylated mixture was approximately 33 %.

The enriched glycosylated Rubusosides were then separated according to their degree of glycosylation via liquid chromatography with a HILIC phase (Uptisphere^{®} Strategytm HIA-HILIC (15 µm, 100 Å, 60 x 226 mm)). The chromatography conditions were as follows:

| **Column** | Uptisphere^{®} Strategytm HIA-HILIC (15 µm, 100 Å, 60 x 226 mm) | | |
|---|---|---|---|
| **Temperature** | Room temperature (20 °C) | | |
| **Mobile Phase** | A: Water | | |
| | B: Acetonitrile | | |

| | Time [min] | A [%] | B [%] |
|---|---|---|---|
| | 0 | 10 | 90 |
| **Gradient** | 101 | 40 | 60 |
| | 121 | 10 | 90 |
| | 141 | 10 | 90 |
| **Flow rate** | 15 ml/min | | |
| **Injection volume** | 15 mL | | |
| **Detection** | UV at 210 nm | | |

The fractions were collected and freeze-dried. The following yields were obtained, when 10.25 g were used:

| **Fraction** | **n-(α)-Glucose** | **Yield [mg]** | **Proportion to weight of sample taken (10.25 g) [%]** |
|---|---|---|---|
| Rubusoside | 0 | 2890.58 | 28 |
| G1 | 1 | 2699.86 | 26 |
| G2 | 2 | 1565.6 | 15 |
| G3 | 3 | 980.5 | 10 |
| G4 | 4 | 610.4 | 6 |
| Sum | | 8746.96 | 85 |

Each fraction was then further separated according to the contained isomers by using liquid chromatography with a RP phase (Phenomenex^{®} Luna C-18, 5 µm, 100 Å, 21.2 x 250 mm). The chromatography conditions were as follows:

| **Column** | Phenomenex^{®} Luna C-18, 5 µm, 100 Å, 21.2 x 250 mm | |
|---|---|---|
| **Temperature** | Room temperature (20 °C) | |
| **Mobile Phase** | A: Water | |
| | B: Acetonitrile | |

| | Fraction | B [%] |
|---|---|---|
| **Elution mode** | G1 | 30 |
| | G2 | 29 |
| | G3 | 30 |
| **Flow rate** | 15 ml/min | |
| **Injection volume** | 400 µL | |
| **Detection** | UV at 210 nm | |

The following isomers were obtained, when 50.1 mg of G1, 1565.6 mg of G2 and 980.5 mg of G3 were used:

| **Fraction** | **Compound** | **n-(α)-Glucose** | **Yield [mg]** | **Proportion to weight of sample taken (50.1 mg** / **1565.6 mg** / **980.5 mg) [%]** |
|---|---|---|---|---|
| 1a | **1** | 1 | 1.8 | 3.6 |
| 1b | **2** | 1 | 0.9 | 1.8 |
| 2a | **3** | 2 | 29.77 | 2.0 |
| 2b | **4** | 2 | 7.38 | 0.5 |
| 2c | **5 and 6** | 2 | 57.37 | 4.0 |
| 3a | **7** | 3 | 9.38 | 1.0 |
| 3b | **8** | 3 | 5.79 | 0.7 |
| 3c | **9 and 10** | 3 | 22.67 | 3.0 |
| 3d | **11** | 3 | 18.48 | 2.0 |

### Example 3: Structural characterization of the obtained isomers

### Example 3.1: NMR

NMR experiments were performed on a Bruker 600 MHz spectrometer. Samples were dissolved in dimethylsulfoxide-d6. Along with the sample signals, signals from dimethylsulfoxide at δ_{H}= 2.50 ppm and δ_{C}= 39.51 ppm were observed. Signals were assigned based on ¹H, ¹³C, HSQC, HMBC, COSY, TOCSY and HSQC-TOCSY experiments. Nomenclature of investigated steviol glycosides was made according to Formula (II).

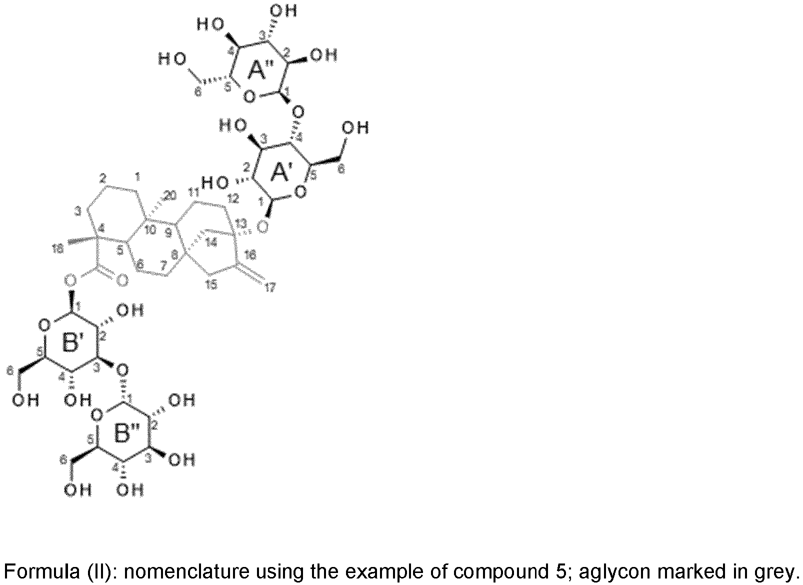

¹H spectrum can be divided into three signal clusters, which covers the ranges of 0-2.5 ppm, 2.5-4 ppm and 4-5.5 ppm. Signals in the range from 0-2.5 ppm can be assigned to the protons of the aglycon backbone, except for the two protons of the exo-methylene group. Signals in the range from 2.5-4 ppm can be assigned to the protons of the attached sugar units. Anomeric protons and protons of the exo-methylene group can be found in the range from 4-5.5 ppm. Based on the different coupling constants of the anomeric protons, α-(J= 3-4 Hz) and β- (J=7-8 Hz) glycosidic bonds can be distinguished.

Starting from position 19, which can be assigned by characteristic chemical shift of carboxyl group, the signals in the immediate vicinity can be determined by combining COSY, HMBC and HSQC experiments. HMBC couplings of the anomeric protons with the corresponding ¹³C signals are used to uniquely identify the linkages of the sugar chains. The sugar sequences were further confirmed by using the combination of HSQC-TOCSY and COSY experiments.

Signals assignments of the NMR experiments described above were summarized in the following tables for each compound.

### Example 3.1.1: Compound 1

| **No** | **Atom Type** | **δ _{H} (Multiplicity, *J*)** | **δ _{C}** | **HMBC** |
|---|---|---|---|---|
| 1a | CH₂ | 0.77 (m) | 40.0 | - |
| 1b | CH₂ | 1.78 (m) | 40.0 | - |
| 2a | CH₂ | 1.37 (m) | 18.7 | 4 |
| 2b | CH₂ | 1.78 (m) | 18.7 | - |
| 3a | CH₂ | 0.98 (m) | 37.1 | - |
| 3b | CH₂ | 2.02 (m) | 37.1 | - |
| 4 | C | - | 43.2 | 2a |
| 5 | CH | 1.05 (m) | 56.3 | 6b, 18, 20 |
| 6a | CH₂ | 1.86 (m) | 21.0 | - |
| 6b | CH₂ | 1.73 (m) | 21.0 | - |
| 7a | CH₂ | 1.48 (m) | 40.9 | - |
| 7b | CH₂ | 1.37 (m) | 40.9 | - |
| 8 | C | - | 41.2 | - |
| 9 | CH | 0.93 (d, 8.2 Hz) | 53.1 | 10, 11b, 15, 20 |
| 10 | C | - | 38.8 | 9 |
| 11a | CH₂ | 1.48 (m) | 19.8 | - |
| 11b | CH₂ | 1.78 (m) | 19.8 | - |
| 12a | CH₂ | 1.39 (m) | 37.4 | - |
| 12b | CH₂ | 1.81 (m) | 37.4 | - |
| 13 | C | - | 85.3 | A'1, 17b, 17a |
| 14a | CH₂ | 1.44 (m) | 43.2 | - |
| 14b | CH₂ | 2.02 (m) | 43.2 | 15, 16 |
| 15 | CH₂ | 2.02 (m) | 47.3 | 16 |
| 16 | C | - | 152.5 | 14b, 15, 17a |
| 17a | CH₂ | 5.14 (brs) | 104.4 | 13, 15, 16 |
| 17b | CH₂ | 4.77 (br s) | 104.4 | 13, 15 |
| 18 | CH₃ | 1.14 (s) | 28.2 | 5, 19 |
| 19 | C | - | 175.2 | B'1, 18 |
| 20 | CH₃ | 0.84 (s) | 15.4 | 5, 9 |
| A'1 | CH | 4.29 (d, 7.8 Hz) | 97.8 | 13 |
| A'2 | CH | 2.92 (m) | 73.6 | - |
| A'3 | CH | 3.13 (m) | 76.8 | - |
| A'4 | CH | 3.05 (m) | 70.1 | - |
| A'5 | CH | 2.98 (m) | 76.4 | - |
| A'6a | CH₂ | 3,44 (m)^{¥} | 60.2-61.0^{¥} | - |
| A'6b | CH₂ | 3,62 (m)^{¥} | 60.2-61.0^{¥} | - |
| B'1 | CH | 5.35 (d, 8.1 Hz) | 93.8 | 19 |
| B'2 | CH | 3.29 (m) | 71.0 | - |
| B'3 | CH | 3.44 (m) | 85.5 | - |
| B'4 | CH | 3.43 (m) | 68.7 | - |
| B'5 | CH | 3.28 (m) | 76.8 | - |
| B'6a | CH₂ | 3,44 (m)^{¥} | 60.2 - 61.0^{¥} | - |
| B'6b | CH₂ | 3,62 (m)^{¥} | 60.2 - 61.0^{¥} | - |
| B"1 | CH | 5.02 (d, 3.8 Hz) | 99.4 | B'3 |
| B"2 | CH | 3.24 (m) | 72.4 | - |
| B"3 | CH | 3.44 (m) | 73.1 | - |
| B"4 | CH | 3.1 (m) | 70.1 | - |
| B"5 | CH | 3.73 (m) | 72.7 | - |
| B"6a | CH₂ | 3,44 (m)^{¥} | 60.2 - 61.0^{¥} | - |
| B"6b | CH₂ | 3,62 (m)^{¥} | 60.2 - 61.0^{¥} | - |

| | | | | |
|---|---|---|---|---|
| ^{¥} signals could not be unequivocally assigned due to close chemical shits or overlapping resonances | | | | |

### Example 3.1.2: Compound 2

| **No** | **Atom Type** | **δ _{H} (Multiplicity, *J*)** | **δ _{C}** | **HMBC** |
|---|---|---|---|---|
| 1a | CH₂ | 0,77 (m) | 39.8 | 20 |
| 1b | CH₂ | 1,78 (m) | 39.8 | - |
| 2a | CH₂ | 1.35 (m) | 18.6 | - |
| 2b | CH₂ | 1.77 (m) | 18.6 | - |
| 3a | CH₂ | 0.98 (m) | 37.2 | - |
| 3b | CH₂ | 2.08 (m) | 37.2 | - |
| 4 | C | - | 43.2 | 18 |
| 5 | CH | 1.05 (m) | 56.3 | 18 |
| 6a | CH₂ | 1.91 (m) | 21.1 | - |
| 6b | CH₂ | 1.70 (m) | 21.1 | - |
| 7a | CH₂ | 1.35 (m) | 40.7 | - |
| 7b | CH₂ | 1.47 (m) | 40.7 | - |
| 8 | C | - | 41.5 | 9 |
| 9 | CH | 0.92 (d, 8.2 Hz) | 53 | 8, 20 |
| 10 | C | - | 38.8 | - |
| 11a | CH₂ | 1.5 (m) | 19.8 | - |
| 11b | CH₂ | 1.69 (m) | 19.8 | - |
| 12a | CH₂ | 1.37 (m) | 36.8 | - |
| 12b | CH₂ | 1.85 (m) | 36.8 | - |
| 13 | C | - | 85.1 | 17a, A'1 |
| 14a | CH₂ | 1.45 (m) | 43.2 | - |
| 14b | CH₂ | 2.09 (m) | 43.2 | - |
| 15 | CH₂ | 2.01 (m) | 47.2 | - |
| 16 | C | - | 152.8 | - |
| 17a | CH₂ | 4.76 (br s) | 104.1 | 13, 15 |
| 17b | CH₂ | 5.09 (br s) | 104.1 | 15 |
| 18 | CH₃ | 1.14 (s) | 28.1 | 3b, 4,5, 19 |
| 19 | C | - | 175.5 | 18, B'1 |
| 20 | CH₃ | 0.86 (s) | 15.2 | 5, 9 |
| A'1 | CH | 4.38 (d, 7.7 Hz) | 97.7 | 13 |
| A'2 | CH | 3.07 (m) | 72.1 | - |
| A'3 | CH | 3.31 (m) | 86.5 | - |
| A'4 | CH | 3.31 (m) | 69.7 | - |
| A'5 | CH | 3.08 (m) | 75.8 | - |
| A'6a | CH₂ | 3.44 (m)^{¥} | 60.5 - 60.7^{¥} | - |
| A'6b | CH₂ | 3.62 (m)^{¥} | 60.5 - 60.7^{¥} | - |
| A"1 | CH | 4.96 (d, 3.9 Hz) | 99.9 | A'3 |
| A"2 | CH | 3.22 (m) | 72.4 | - |
| A"3 | CH | 3.42 (m) | 73.4 | - |
| A"4 | CH | 3.1 (m) | 70 | - |
| A"5 | CH | 3.72 (m) | 72.6 | - |
| A"6a | CH₂ | 3.44 (m)^{¥} | 60.5 - 60.7^{¥} | - |
| A"6b | CH₂ | 3.62 (m)^{¥} | 60.5 - 60.7^{¥} | - |
| B'1 | CH | 5.27 (d, 8.2 Hz) | 94 | 19 |
| B'2 | CH | 3.15 (m) | 72.5 | - |
| B'3 | CH | 3.22 (m) | 76.8 | - |
| B'4 | CH | 3.13 (m) | 69.5 | - |
| B'5 | CH | 3.18 (m) | 77.6 | - |
| B'6a | CH₂ | 3.44 (m)^{¥} | 60.5 - 60.7^{¥} | - |
| B'6b | CH₂ | 3.62 (m)^{¥} | 60.5 - 60.7^{¥} | - |

| | | | | |
|---|---|---|---|---|
| ^{¥} signals could not be unequivocally assigned due to close chemical shits or overlapping resonances | | | | |

### Example 3.1.3: Compound 3

| **No** | **Atom Type** | **δ _{H} (Multiplicity, *J*)** | **δ _{C}** | **HMBC** |
|---|---|---|---|---|
| 1a | CH₂ | 1.70 (m) | 39.9 | - |
| 1b | CH₂ | 0.70 (m) | 39.9 | - |
| 2a | CH₂ | 1.70 (m) | 18.9 | - |
| 2b | CH₂ | 1.30 (m) | 18.9 | - |
| 3a | CH₂ | 2.09 (m) | 37.0 | - |
| 3b | CH₂ | 0.91 (m) | 37.0 | - |
| 4 | C | - | 43.4 | 5, 9, 18 |
| 5 | CH | 0.97 (m) | 56.5 | 4, 9, 10, 18, 19, 20 |
| 6 | CH₂ | 1.70 (m) | 21.1 | 5 |
| 7a | CH₂ | 1.41 (m) | 41.0 | - |
| 7b | CH₂ | 1.30 (m) | 41.0 | - |
| 8 | C | - | 41.3 | - |
| 9 | CH | 0.87 (m) | 53.1 | 10, 12b, 14b, 20 |
| 10 | C | - | 38.9 | 5, 9, 20 |
| 11a | CH₂ | 1.70 (m) | 19.9 | - |
| 11b | CH₂ | 1.41 (m) | 19.9 | - |
| 12a | CH₂ | 1.70 (m) | 37.4 | - |
| 12b | CH₂ | 1.30 (m) | 37.4 | - |
| 13 | C | - | 85.4 | A'1, 17a, 17b |
| 14a | CH₂ | 1.96 (m) | 43.4 | 15 |
| 14b | CH₂ | 1.41 (m) | 43.4 | - |
| 15 | CH₂ | 1.96 (m) | 47.5 | - |
| 16 | C | - | 152.6 | 17a |
| 17a | CH₂ | 5.08 (br s) | 104.4 | 13, 15, 16 |
| 17b | CH₂ | 4.70 (br s) | 104.4 | 13, 15 |
| 18 | CH₃ | 1.08 (s) | 28.4 | 3b, 4, 5, 19 |
| 19 | C | - | 175.1 | 5,18, B'1 |
| 20 | CH₃ | 0.77 (s) | 15.7 | 1b, 5, 9, 10 |
| A'1 | CH | 4.22 (d, 7.8 Hz) | 97.8 | 13 |
| A'2 | CH | 2.86 (m) | 73.7 | - |
| A'3 | CH | 3.06 (m) | 76.9 | - |
| A'4 | CH | 2.99 (m) | 70.2 | - |
| A'5 | CH | 2.91 (m) | 76.6 | - |
| A'6 | CH₂ | 3.35 (m)^{¥} | 60.1 - 61.1^{¥} | - |
| A'6 | CH₂ | 3.55 (m)^{¥} | 60.1 - 61.1^{¥} | - |
| B'1 | CH | 5.27 (d, 8.1 Hz) | 93.9 | 19 |
| B'2 | CH | 3.35 (m) | 70.9 | - |
| B'3 | CH | 3.35 (m) | 84.9 | - |
| B'4 | CH | 3.35 (m) | 69.2 | - |
| B'5 | CH | 3.35 (m) | 77.0 | - |
| B'6a | CH₂ | 3.35 (m)^{¥} | 60.1 - 61.1^{¥} | - |
| B'6b | CH₂ | 3.55 (m)^{¥} | 60.1 - 61.1^{¥} | - |
| B"1 | CH | 5.01 (d, 3.9 Hz) | 99.2 | B'3 |
| B"2 | CH | 3.35 (m) | 72.0 | - |
| B"3 | CH | 3.65 (m) | 73.2 | - |
| B"4 | CH | 3.35 (m) | 79.7 | - |
| B"5 | CH | 3.80 (m) | 70.9 | - |
| B"6a | CH₂ | 3.35 (m)^{¥} | 60.1 - 61.1^{¥} | - |
| B"6b | CH₂ | 3.55 (m)^{¥} | 60.1 - 61.1^{¥} | - |
| B‴1 | CH | 5.94 (d, 3.75 Hz) | 100.8 | B"4 |
| B‴2 | CH | 3.16 (m) | 72.6 | - |
| B‴3 | CH | 3.35 (m) | 73.3 | - |
| B‴4 | CH | 2.99 (m) | 69.9 | - |
| B‴5 | CH | 3.35 (m) | 73.5 | - |
| B‴6a | CH₂ | 3.35 (m)^{¥} | 60.1 - 61.1^{¥} | - |
| B‴6b | CH₂ | 3.55 (m)^{¥} | 60.1 - 61.1^{¥} | - |

| | | | | |
|---|---|---|---|---|
| ^{¥} signals could not be unequivocally assigned due to close chemical shits or overlapping resonances | | | | |

### Example 3.1.4: Compound 4

| **No** | **Atom Type** | **δ _{H} (Multiplicity, *J*)** | **δ _{C}** | **HMBC** |
|---|---|---|---|---|
| 1a | CH₂ | 1.79 (m) | 39.9 | - |
| 1b | CH₂ | 0.77 (m) | 39.9 | - |
| 2a | CH₂ | 1.79 (m) | 18.7 | - |
| 2b | CH₂ | 1.35 (m) | 18.7 | - |
| 3a | CH₂ | 2.12 (m) | 37.1 | - |
| 3b | CH₂ | 0.98 (m) | 37.1 | - |
| 4 | C | - | 43.2 | - |
| 5 | CH | 1.04 (m) | 56.3 | 18, 19, 20 |
| 6 | CH₂ | 1.79 (m) | 21.0 | 5 |
| 7a | CH₂ | 1.47 (m) | 40.9 | 5, 9 |
| 7b | CH₂ | 1.35 (m) | 40.9 | - |
| 8 | C | - | 41.3 | - |
| 9 | CH | 0.93 (m) | 53.0 | 11b, 20 |
| 10 | C | - | 38.8 | 20 |
| 11a | CH₂ | 1.79 (m) | 19.8 | - |
| 11b | CH₂ | 1.47 (m) | 19.8 | - |
| 12a | CH₂ | 1.79 (m) | 37.3 | - |
| 12b | CH₂ | 1.35 (m) | 37.3 | - |
| 13 | C | - | 85.4 | - |
| 14a | CH₂ | 2.03 (m) | 43.1 | 15b, 16 |
| 14b | CH₂ | 1.47 (m) | 43.1 | 9 |
| 15a | CH₂ | 2.06 (m) | 47.3 | 9 |
| 15b | CH₂ | 1.99 (m) | 47.3 | - |
| 16 | C | - | 152.5 | 14a |
| 17a | CH₂ | 5.13 (brs) | 104.4 | 15b |
| 17b | CH₂ | 4.77 (br s) | 104.4 | 15b |
| 18 | CH₃ | 1.14 (s) | 28.2 | 5, 19 |
| 19 | C | - | 175.1 | 5, 18, B'1 |
| 20 | CH₃ | 0.83 (s) | 15.4 | 5, 9, 10 |
| A'1 | CH | 4.38 (d, 7.7 Hz) | 97.5 | 13 |
| A'2 | CH | 3.09 (m) | 72.2 | - |
| A'3 | CH | 3.39 (m) | 86.6 | - |
| A'4 | CH | 3.39 (m) | 69.3 | - |
| A'5 | CH | 3.09 (m) | 75.8 | - |
| A'6a | CH₂ | 3.39 (m)^{¥} | 60.2 - 60.7^{¥} | - |
| A'6b | CH₂ | 3.61 (m)^{¥} | 60.2 - 60.7^{¥} | - |
| A"1 | CH | 4.95 (d, 3.8 Hz) | 99.9 | A'3 |
| A"2 | CH | 3.39 (m) | 72.4 | - |
| A"3 | CH | 3.39 (m) | 73.3, 73.4^{¥} | - |
| A"4 | CH | 3.09 (m) | 70.0 | - |
| A"5 | CH | 3.71 (m) | 72.7, 72.7^{¥} | - |
| A"6a | CH₂ | 3.39 (m)^{¥} | 60.2 - 60.7^{¥} | - |
| A"6b | CH₂ | 3.61 (m)^{¥} | 60.2 - 60.7^{¥} | - |
| B'1 | CH | 5.34 (d, 8.0 Hz) | 93.8 | 19 |
| B'2 | CH | 3.39 (m) | 71.0 | - |
| B'3 | CH | 3.39 (m) | 85.6 | - |
| B'4 | CH | 3.39 (m) | 68.7 | - |
| B'5 | CH | 3.39 (m) | 76.8 | - |
| B'6a | CH₂ | 3.39 (m)^{¥} | 60.2 - 60.7^{¥} | - |
| B'6b | CH₂ | 3.61 (m)^{¥} | 60.2 - 60.7^{¥} | - |
| B"1 | CH | 5.02 (d, 3.8 Hz) | 99.5 | B'3 |
| B"2 | CH | 3.39 (m) | 72.2 | - |
| B"3 | CH | 3.39 (m) | 73.3, 73.4^{¥} | - |
| B"4 | CH | 3.09 (m) | 69.9 | - |
| B"5 | CH | 3.71 (m) | 72.7, 72.7^{¥} | - |
| B"6a | CH₂ | 3.39 (m)^{¥} | 60.2 - 60.7^{¥} | - |
| B"6b | CH₂ | 3.61 (m)^{¥} | 60.2 - 60.7^{¥} | - |

| | | | | |
|---|---|---|---|---|
| ^{¥} signals could not be unequivocally assigned due to close chemical shits or overlapping resonances | | | | |

### Example 3.1.5: Compound 5

| **No** | **Atom Type** | **δH (Multiplicity, *J*)** | **δ _{C}** | **HMBC ........** |
|---|---|---|---|---|
| 1a | CH₂ | 1.76 (m) | 39.9 | - |
| 1b | CH₂ | 0.78 (m) | 39.9 | 20 |
| 2a | CH₂ | 1.76 (m) | 18.8 | - |
| 2b | CH₂ | 1.35 (m) | 18.8 | - |
| 3a | CH₂ | 2.05 (m) | 37.1 | - |
| 3b | CH₂ | 0.98 (m) | 37.1 | 5, 19 |
| 4 | C | - | 43.2 | - |
| 5 | CH | 1.04 (m) | 56.3 | 3b, 18 |
| 6 | CH₂ | 1.76 (m) | 21.0 | - |
| 7a | CH₂ | 1.48 (m) | 40.9 | 9 |
| 7b | CH₂ | 1.35 (m) | 40.9 | - |
| 8 | C | - | 41.2 | - |
| 9 | CH | 0.93 (m) | 53.1 | 7a, 11b, 14b, 14a, 15, 20 |
| 10 | C | - | 38.8 | 20 |
| 11a | CH₂ | 1.76 (m) | 19.8 | - |
| 11b | CH₂ | 1.48 (m) | 19.8 | 9 |
| 12a | CH₂ | 1.76 (m) | 37.5 | - |
| 12b | CH₂ | 1.35 (m) | 37.5 | - |
| 13 | C | - | 85.5 | A'1 |
| 14a | CH₂ | 2.05 (m) | 43.1 | 9 |
| 14b | CH₂ | 1.48 (m) | 43.1 | 9 |
| 15 | CH₂ | 2.05 (m) | 47.4 | 9 |
| 16 | C | - | 152.4 | - |
| 17a | CH₂ | 5.13 (br s) | 104.4 | 15 |
| 17b | CH₂ | 4.77 (br s) | 104.4 | 15 |
| 18 | CH₃ | 1.15 (s) | 28.3 | 5, 19 |
| 19 | C | - | 175.1 | 3b, 5a, 18, B'1 |
| 20 | CH₃ | 0.84 (s) | 15.4 | 9, 10 |
| A'1 | CH | 4.34 (d, 7.8 Hz) | 97.6 | 13 |
| A'2 | CH | 2.98 (m) | 73.2 | - |
| A'3 | CH | 3.41 (m) | 76.2 | - |
| A'4 | CH | 3.34 (m) | 79.5 | - |
| A'5 | CH | 3.13 (m) | 74.9 | - |
| A'6a | CH₂ | 3.45 (m)^{¥} | 60.2 - 60.7^{¥} | - |
| A'6b | CH₂ | 3.62 (m)^{¥} | 60.2 - 60.7^{¥} | - |
| A"1 | CH | 5.00 (d, 4.1 Hz) | 100.5 | A'4 |
| A"2 | CH | 3.22 (m) | 72.4 | - |
| A"3 | CH | 3.45 (m) | 73.3, 73.4^{¥} | - |
| A"4 | CH | 3.06 (m) | 69.7 | - |
| A"5 | CH | 3.34 (m) | 73.2 | - |
| A"6a | CH₂ | 3.45 (m)^{¥} | 60.2 - 60.7^{¥} | - |
| A"6b | CH₂ | 3.62 (m)^{¥} | 60.2 - 60.7^{¥} | - |
| B'1 | CH | 5.35 (d, 8.1 Hz) | 93.7 | 19 |
| B'2 | CH | 3.34 (m) | 71.0 | - |
| B'3 | CH | 3.43 (m) | 85.7 | - |
| B'4 | CH | 3.45 (m) | 68.7 | - |
| B'5 | CH | 3.34 (m) | 76.9 | - |
| B'6a | CH₂ | 3.45 (m)^{¥} | 60.2 - 60.7^{¥} | - |
| B'6b | CH₂ | 3.62 (m)^{¥} | 60.2 - 60.7^{¥} | - |
| B"1 | CH | 5.01 (d, 4.7 Hz) | 99.6 | B'3 |
| B"2 | CH | 3.22 (m) | 72.3 | - |
| B"3 | CH | 3.45 (m) | 73.3, 73.4^{¥} | - |
| B"4 | CH | 3.13 (m) | 70.0 | - |
| B"5 | CH | 3.72 (m) | 72.7 | - |
| B"6a | CH₂ | 3.45 (m)^{¥} | 60.2 - 60.7^{¥} | - |
| B"6b | CH₂ | 3.62 (m)^{¥} | 60.2 - 60.7^{¥} | - |

| | | | | |
|---|---|---|---|---|
| ^{¥} signals could not be unequivocally assigned due to close chemical shits or overlapping resonances | | | | |

### Example 3.1.6: Compound 7

| **No** | **Atom Type** | **δ _{H} (Multiplicity, *J*)** | **δ _{C}** | **HMBC** |
|---|---|---|---|---|
| 1a | CH₂ | 1.77 (m) | 39.9 | - |
| 1b | CH₂ | 0.77 (m) | 39.9 | - |
| 2a | CH₂ | 1.77 (m) | 18.8 | - |
| 2b | CH₂ | 1.36 (m) | 18.8 | - |
| 3a | CH₂ | 2.17 (m) | 36.8 | - |
| 3b | CH₂ | 0.97 (m) | 36.8 | 19 |
| 4 | C | - | 43.3 | - |
| 5 | CH | 1.04 (m) | 56.3 | 6, 18, 20 |
| 6 | CH₂ | 1.77 (m) | 21.0 | 5 |
| 7a | CH₂ | 1.47 (m) | 40.9 | 5, 9 |
| 7b | CH₂ | 1.36 (m) | 40.9 | - |
| 8 | C | - | 41.2 | - |
| 9 | CH | 0.93 (m) | 53.0 | 7a, 11b, 14b,20 |
| 10 | C | - | 38.8 | 5, 9, 20 |
| 11a | CH₂ | 1.77 (m) | 19.8 | - |
| 11b | CH₂ | 1.47 (m) | 19.8 | 9, 12b, 13 |
| 12a | CH₂ | 1.77 (m) | 37.4 | - |
| 12b | CH₂ | 1.36 (m) | 37.4 | - |
| 13 | C | - | 85.4 | A'1, 14b, 14a, 17b, 17a |
| 14a | CH₂ | 2.02 (m) | 43.2 | 13, 15, 16 |
| 14b | CH₂ | 1.47 (m) | 43.2 | 9, 13 |
| 15 | CH₂ | 2.02 (m) | 47.3 | 16 |
| 16 | C | - | 152.4 | 15, 17a |
| 17a | CH₂ | 5.14 (brs) | 104.4 | 13, 15, 16 |
| 17b | CH₂ | 4.77 (br s) | 104.4 | 13, 15 |
| 18 | CH₃ | 1.15 (s) | 72.0 | 5, 19 |
| 19 | C | - | 174.9 | 3b, 5, 18, B'1 |
| 20 | CH₃ | 0.83 (s) | 15.6 | 1b, 5, 9, 10 |
| A'1 | CH | 4.29 (d, 7.8 Hz) | 97.8 | 13 |
| A'2 | CH | 2.92 (m) | 73.6 | A'1 |
| A'3 | CH | 3.13 (m) | 76.7 | - |
| A'4 | CH | 3.04 (m) | 70.0 | - |
| A'5 | CH | 2.98 (m) | 76.5 | - |
| A'6a | CH₂ | 3.45 (m)^{¥} | 60.0 - 60.9^{¥} | - |
| A'6b | CH₂ | 3.62 (m)^{¥} | 60.0 - 60.9^{¥} | - |
| B'1 | CH | 5.34 (d, 8.1 Hz) | 93.7 | 19 |
| B'2 | CH | 3.38 (m) | 70.8 | - |
| B'3 | CH | 3.44 (m) | 84.3 | - |
| B'4 | CH | 3.38 (m) | 69.1 | - |
| B'5 | CH | 3.38 (m) | 76.9 | - |
| B'6a | CH₂ | 3.45 (m)^{¥} | 60.0 - 60.9^{¥} | - |
| B'6b | CH₂ | 3.62 (m)^{¥} | 60.0 - 60.9^{¥} | - |
| B"1 | CH | 5.08 (d, 3.8 Hz) | 99.1 | B'3 |
| B"2 | CH | 3.38 (m) | 71.8 | - |
| B"3 | CH | 3.72 (m) | 72.9 | - |
| B"4 | CH | 3.38 (m) | 79.9 | - |
| B"5 | CH | 3.88 (m) | 70.7 | - |
| B"6a | CH₂ | 3.45 (m)^{¥} | 60.0 - 60.9^{¥} | - |
| B"6b | CH₂ | 3.62 (m)^{¥} | 60.0 - 60.9^{¥} | - |
| B‴1 | CH | 5.00 (d, 3.7 Hz) | 100.6, 100.7 | B"4 |
| B‴2 | CH | 3.38 (m) | 71.6 | - |
| B‴3 | CH | 3.64 (m) | 73.1 | - |
| B‴4 | CH | 3.38 (m) | 79.3 | - |
| B‴5 | CH | 3.64 (m) | 72.0 | - |
| B‴6a | CH₂ | 3.45 (m)^{¥} | 60.0 - 60.9^{¥} | - |
| B‴6b | CH₂ | 3.62 (m)^{¥} | 60.0 - 60.9^{¥} | - |
| B^{IV}1 | CH | 5.00 (d, 3.7 Hz) | 100.6, 100.7 | B‴4 |
| B^{IV}2 | CH | 3.38 (m) | 73.2 | - |
| B^{IV}3 | CH | 3.38 (m) | 73.4 | - |
| B^{IV}4 | CH | 3.38 (m) | 69.7 | - |
| B^{IV}5 | CH | 3.23 (m) | 72.4 | - |
| B^{IV}6a | CH₂ | 3.45 (m)^{¥} | 60.0 - 60.9^{¥} | - |
| B^{IV}6b | CH₂ | 3.62 (m)^{¥} | 60.0 - 60.9^{¥} | - |

| | | | | |
|---|---|---|---|---|
| ^{¥} signals could not be unequivocally assigned due to close chemical shits or overlapping resonances | | | | |

### Example 3.1.7: Compound 8

| **No** | **Atom Type** | **δ _{H} (Multiplicity, J)** | **δ _{C}** | **HMBC** |
|---|---|---|---|---|
| 1a | CH₂ | 0.78 (m) | 40.0 | - |
| 1b | CH₂ | 1.76 (m) | 40.0 | - |
| 2a | CH₂ | 1.36 (m) | 18.8 | - |
| 2b | CH₂ | 1.76 (m) | 18.8 | - |
| 3a | CH₂ | 2.17 (m) | 36.9 | - |
| 3b | CH₂ | 0.98 (m) | 36.9 | - |
| 4 | C | - | 43.3 | - |
| 5 | CH | 1.04 (m) | 56.4 | 18 |
| 6 | CH₂ | 1.76 (m) | 21.0 | - |
| 7a | CH₂ | 1.36 (m) | 40.9 | - |
| 7b | CH₂ | 1.46 (m) | 40.9 | - |
| 8 | C | - | 41.3 | - |
| 9 | CH | 0.94 (m) | 53.0 | - |
| 10 | C | - | 38.8 | 9, 20 |
| 11a | CH₂ | 1.46 (m) | 19.8 | - |
| 11b | CH₂ | 1.76 (m) | 19.8 | - |
| 12a | CH₂ | 1.36 (m) | 37.3 | - |
| 12b | CH₂ | 1.76 (m) | 37.3 | - |
| 13 | C | - | 85.5 | A'1 |
| 14a | CH₂ | 2.02 (m) | 43.2 | - |
| 14b | CH₂ | 1.46 (m) | 43.2 | - |
| 15 | CH₂ | 2.02 (m) | 47.3 | - |
| 16 | C | - | 152.4 | - |
| 17a | CH₂ | 5.14 (brs) | 104.4 | 15 |
| 17b | CH₂ | 4.78 (br s) | 104.4 | 15 |
| 18 | CH₃ | 1.15 (s) | 28.4 | 5, 19 |
| 19 | C | - | 175.0 | B'1, 5, 18 |
| 20 | CH₃ | 0.83 (s) | 15.6 | 10 |
| A'1 | CH | 4.38 (d, 7.7 Hz) | 97.6 | 13 |
| A'2 | CH | 3.09 (m) | 72.3 | - |
| A'3 | CH | 3.32 (m) | 86.5 | - |
| A'4 | CH | 3.32 (m) | 69.4 | - |
| A'5 | CH | 3.09 (m) | 75.7 | - |
| A'6a | CH₂ | 3.45 (m)^{¥} | 60.0 - 60.7^{¥} | - |
| A'6b | CH₂ | 3.62 (m)^{¥} | 60.0 - 60.7^{¥} | - |
| A"1 | CH | 4.96 (d, 3.8 Hz) | 99.9 | A'3 |
| A"2 | CH | 3.23 (m) | 72.5 | - |
| A"3 | CH | 3.32 (m)^{¥} | 71.8 -73.4^{¥} | - |
| A"4 | CH | 3.09 (m) | 70.0 | - |
| A"5 | CH | 3.70 (m) | 72.6 | - |
| A"6a | CH₂ | 3.45 (m)^{¥} | 60.0 - 60.7^{¥} | - |
| A"6b | CH₂ | 3.62 (m)^{¥} | 60.0 - 60.7^{¥} | - |
| B'1 | CH | 5.34 (d, 8.1 Hz) | 93.7 | 19 |
| B'2 | CH | 3.36 (m) | 70.9 | - |
| B'3 | CH | 3.45 (m) | 84.9 | - |
| B'4 | CH | 3.45 (m) | 69.0 | - |
| B'5 | CH | 3.09 (m) | 70.1 | - |
| B'6a | CH₂ | 3.45 (m)^{¥} | 60.0 - 60.7^{¥} | - |
| B'6b | CH₂ | 3.62 (m)^{¥} | 60.0 - 60.7^{¥} | - |
| B"1 | CH | 5.07 (d, 3.8 Hz) | 99.2 | B'3 |
| B"2 | CH | 3.36 (m) | 72.2 | - |
| B"3 | CH | 3.36 (m)^{¥} | 71.8 -73.4^{¥} | - |
| B"4 | CH | 3.36 (m) | 79.6 | - |
| B"5 | CH | 3.36 (m)^{¥} | 71.8 -73.4^{¥} | - |
| B"6a | CH₂ | 3.45 (m)^{¥} | 60.0 - 60.7^{¥} | - |
| B"6b | CH₂ | 3.62 (m)^{¥} | 60.0 - 60.7^{¥} | - |
| B‴1 | CH | 5.00 (d, 3.8 Hz) | 100.8 | B"4 |
| B‴2 | CH | 3.23 (m) | 72.4 | - |
| B‴3 | CH | 3.36 (m)^{¥} | 71.8 -73.4^{¥} | - |
| B‴4 | CH | 3.09 (m)^{¥} | 69.7 - 69.9^{¥} | - |
| B‴5 | CH | 3.36 (m)^{¥} | 71.8 -73.4^{¥} | - |
| B‴6a | CH₂ | 3.45 (m)^{¥} | 60.0 - 60.7^{¥} | - |
| B‴6b | CH₂ | 3.62 (m)^{¥} | 60.0 - 60.7^{¥} | - |

| | | | | |
|---|---|---|---|---|
| ^{¥} signals could not be unequivocally assigned due to close chemical shits or overlapping resonances | | | | |

### Example 3.1.8: Compound 9

| **No** | **Atom Type** | **δ _{H} (Multiplicity, *J*)** | **δ _{C}** | **HMBC** |
|---|---|---|---|---|
| 1a | CH₂ | 1.77 (m) | 40.1 | - |
| 1b | CH₂ | 0.78 (m) | 40.1 | - |
| 2a | CH₂ | 1.77 (m) | 19.0 | - |
| 2b | CH₂ | 1.36 (m) | 19.0 | - |
| 3a | CH₂ | 2.17 (m) | 37.0 | - |
| 3b | CH₂ | 0.97 (m) | 37.0 | - |
| 4 | C | - | 43.4 | - |
| 5 | CH | 1.03 (m) | 56.5 | 18, 20 |
| 7a | CH₂ | 1.47 (m) | 41.0 | 9 |
| 7b | CH₂ | 1.36 (m) | 41.0 | - |
| 8 | C | - | 41.3 | - |
| 9 | CH | 0.94 (m) | 53.1 | 7a, 11b, 14b,20 |
| 10 | C | - | 38.9 | 9, 20 |
| 11a | CH₂ | 1.77 (m) | 19.9 | - |
| 11b | CH₂ | 1.47 (m) | 19.9 | 9, 13 |
| 12a | CH₂ | 1.77 (m) | 37.5 | - |
| 12b | CH₂ | 1.40 (m) | 37.5 | - |
| 13 | C | - | 85.6 | 14b, 17b, 17a, A'1 |
| 14a | CH₂ | 2.05 (m) | 43.3 | 15b |
| 14b | CH₂ | 1.47 (m) | 43.3 | 9, 13 |
| 15a | CH₂ | 2.05 (m) | 47.5 | - |
| 15b | CH₂ | 1.99 (m) | 47.5 | 16 |
| 16 | C | - | 152.5 | 15b |
| 17a | CH₂ | 5.13 (brs) | 104.5 | 13, 15b |
| 17b | CH₂ | 4.77 (br s) | 104.5 | 13, 15b |
| 18 | CH₃ | 1.15 (s) | 28.5 | 5, 19 |
| 19 | C | - | 175.0 | 5, 18, B'1 |
| 20 | CH₃ | 0.83 (s) | 15.8 | 5, 9,10 |
| A'1 | CH | 4.34 (d, 7.7 Hz) | 97.7 | 13 |
| A'2 | CH | 2.98 (m) | 73.1 - 73.5^{¥} | - |
| A'3 | CH | 3.43 (m) | 76.3 | - |
| A'4 | CH | 3.31 (m) | 79.4 | - |
| A'5 | CH | 3.64 (m)^{¥} | 73.1 - 73.5^{¥} | - |
| A'6a | CH₂ | 3.47 (m)^{¥} | 60.1 - 60.7^{¥} | - |
| A'6b | CH₂ | 3.63 (m)^{¥} | 60.1 - 60.7^{¥} | - |
| A"1 | CH | 5.00 (m) | 100.6 | A'4 |
| A"2 | CH | 3.23 (m)^{¥} | 72.5-72.6^{¥} | - |
| A"3 | CH | 3.37 (m)^{¥} | 73.1 - 73.5^{¥} | - |
| A"4 | CH | 3.07 (m) | 69.8 - 69.9^{¥} | - |
| A"5 | CH | 3.48 (m)^{¥} | 73.1 - 73.5^{¥} | - |
| A"6a | CH₂ | 3.47 (m)^{¥} | 60.1 - 60.7^{¥} | - |
| A"6b | CH₂ | 3.63 (m)^{¥} | 60.1 - 60.7^{¥} | - |
| B'1 | CH | 5.35 (d, 8.1 Hz) | 93.8 | 19 |
| B'2 | CH | 3.23 (m)^{¥} | 72.5-72.6^{¥} | - |
| B'3 | CH | 3.44 (m) | 84.9 | - |
| B'4 | CH | 3.44 (m) | 69.2 | - |
| B'5 | CH | 3.71 (m)^{¥} | 73.1 - 73.5^{¥} | - |
| B'6a | CH₂ | 3.47 (m)^{¥} | 60.1 - 60.7^{¥} | - |
| B'6b | CH₂ | 3.63 (m)^{¥} | 60.1 - 60.7^{¥} | - |
| B"1 | CH | 5.07 (d, 3.8 Hz) | 99.4 | B'3 |
| B"2 | CH | 3.27 (m)^{¥} | 72.0-72.2^{¥} | - |
| B"3 | CH | 3.37 (m)^{¥} | 72.5-72.6^{¥} | - |
| B"4 | CH | 3.37 (m) | 79.6 | - |
| B"5 | CH | 3.45 (m)^{¥} | 73.1 - 73.5^{¥} | - |
| B"6a | CH₂ | 3.47 (m)^{¥} | 60.1 - 60.7^{¥} | - |
| B"6b | CH₂ | 3.63 (m)^{¥} | 60.1 - 60.7^{¥} | - |
| B‴1 | CH | 5.00 (m) | 100.8 | B"4 |
| B‴2 | CH | 3.27 (m)^{¥} | 72.0-72.2^{¥} | - |
| B‴3 | CH | 3.37 (m)^{¥} | 72.5-72.6^{¥} | - |
| B‴4 | CH | 3.06 (m)^{¥} | 69.8 - 69.9^{¥} | - |
| B‴5 | CH | 3.45 (m)^{¥} | 73.1 - 73.5^{¥} | - |
| B‴6a | CH₂ | 3.47 (m)^{¥} | 60.1 - 60.7^{¥} | - |
| B‴6b | CH₂ | 3.63 (m)^{¥} | 60.1 - 60.7^{¥} | - |

| | | | | |
|---|---|---|---|---|
| ^{¥} signals could not be unequivocally assigned due to close chemical shits or overlapping resonances | | | | |

### Example 3.1.9: Compound 11

| **No** | **Atom Type** | **δ _{H} (muftiplicity, *J*)** | **δ _{C}** | **HMBC** |
|---|---|---|---|---|
| 1a | CH₂ | 1.76 (m) | 40.0 | - |
| 1b | CH₂ | 0.78 (m) | 40.0 | - |
| 2a | CH₂ | 1.76 (m) | 18.8 | - |
| 2b | CH₂ | 1.36 (m) | 18.8 | - |
| 3a | CH₂ | 2.13 (m) | 37.1 | - |
| 3b | CH₂ | 0.98 (m) | 37.1 | - |
| 4 | C | - | 43.3 | - |
| 5 | CH | 1.04 (m) | 56.3 | 18 |
| 6 | CH₂ | 1.76 (m) | 21.0 | - |
| 7a | CH₂ | 1.46 (m) | 40.9 | 9 |
| 7b | CH₂ | 1.36 (m) | 40.9 | - |
| 8 | C | - | 41.2 | - |
| 9 | CH | 0.93 (m) | 53.1 | 7a, 11b, 14b, 20 |
| 10 | C | - | 38.8 | 5, 9, 20 |
| 11a | CH₂ | 1.76 (m) | 19.8 | - |
| 11b | CH₂ | 1.46 (m) | 19.8 | 9 |
| 12a | CH₂ | 1.76 (m) | 37.5 | - |
| 12b | CH₂ | 1.41 (m) | 37.5 | - |
| 13 | C | - | 85.6 | - |
| 14a | CH₂ | 2.00 (m) | 43.2 | 15b, 16 |
| 14b | CH₂ | 1.46 (m) | 43.2 | 9 |
| 15a | CH₂ | 2.06 (m) | 47.5 | 16 |
| 15b | CH₂ | 2.00 (m) | 47.5 | 16 |
| 16 | C | - | 152.3 | 14a, 15b, 15a |
| 17a | CH₂ | 5.15 (br s) | 104.5 | 15b |
| 17b | CH₂ | 4.78 (br s) | 104.5 | 15b |
| 18 | CH₃ | 1.15 (s) | 28.3 | 5, 19 |
| 19 | C | - | 175.0 | B'1, 5, 18 |
| 20 | CH₃ | 0.84 (s) | 15.5 | 9, 10 |
| A'1 | CH | 4.34 (d, 7.7 Hz) | 97.6 | 13 |
| A'2 | CH | 2.98 (m) | 73.2 | - |
| A'3 | CH | 3,43 (m) | 76.3 | - |
| A'4 | CH | 3.33 (m) | 79.7 | - |
| A'5 | CH | 3.64 (m)^{¥} | 73.1 -73.4^{¥} | - |
| A'6a | CH₂ | 3.47 (m)^{¥} | 60.1 - 60.7^{¥} | - |
| A'6b | CH₂ | 3.63 (m)^{¥} | 60.1 - 60.7^{¥} | - |
| A"1 | CH | 5.01 (d, 3.9 Hz) | 100.3 | A'4 |
| A"2 | CH | 3.23 - 3.28 (m)^{¥} | 71.5 - 72.7^{¥} | - |
| A"3 | CH | 3.37 - 3.46 (m)^{¥} | 73.1 -73.3^{¥} | - |
| A"4 | CH | 3.33 (m) | 79.5 | - |
| A"5 | CH | 3.62 (m) | 73.1 - 73.3^{¥} | - |
| A"6a | CH₂ | 3.47 (m)^{¥} | 60.1 - 60.7^{¥} | - |
| A"6b | CH₂ | 3.63 (m)^{¥} | 60.1 - 60.7^{¥} | - |
| A‴1 | CH | 4.99 (d, 3.9 Hz) | 100.7 | A"4 |
| A‴2 | CH | 3.23 - 3.28 (m)^{¥} | 71.5 - 72.7^{¥} | - |
| A‴3 | CH | 3.38 (m) | 73.2 | - |
| A‴4 | CH | 3.06 (m) | 69.8 | - |
| A‴5 | CH | 3.47 (m)^{¥} | 73.1 -73.3^{¥} | - |
| A‴6a | CH₂ | 3.47 (m)^{¥} | 60.1 - 60.7^{¥} | - |
| A‴6b | CH₂ | 3.63 (m)^{¥} | 60.1 - 60.7^{¥} | - |
| B'1 | CH | 5.36 (d, 8.0 Hz) | 93.7 | 19 |
| B'2 | CH | 3.28 (m) | 71.1 | - |
| B'3 | CH | 3.43 (m) | 85.7 | - |
| B'4 | CH | 3.43 (m) | 68.8 | - |
| B'5 | CH | 3.62 (m) | 73.1 - 73.3^{¥} | - |
| B'6a | CH₂ | 3.47 (m)^{¥} | 60.1 - 60.7^{¥} | - |
| B'6b | CH₂ | 3.63 (m)^{¥} | 60.1 - 60.7^{¥} | - |
| B"1 | CH | 5.01 (d, 3.9 Hz) | 99.7 | B'3 |
| B"2 | CH | 3.23 - 3.28 (m)^{¥} | 71.5 - 72.7^{¥} | - |
| B"3 | CH | 3.37 - 3.46 (m)^{¥} | 73.4 | - |
| B"4 | CH | 3.13 (m) | 70.0 | - |
| B"5 | CH | 3.73 (m) | 72.7 | - |
| B"6a | CH₂ | 3.47 (m)^{¥} | 60.1 - 60.7^{¥} | - |
| B"6b | CH₂ | 3.63 (m)^{¥} | 60.1 - 60.7^{¥} | - |

| | | | | |
|---|---|---|---|---|
| ^{¥} signals could not be unequivocally assigned due to close chemical shits or overlapping resonances | | | | |

### Example 3.2: Mass spectrometry

Mass spectrometry for characterizing the structure of the obtained isomers was performed under the following conditions:

| **UPLC** | Waters Aquity I-Class UPLC | | |
|---|---|---|---|
| **Mass spectrometer** | Waters VION IMS QTof | | |
| **Column** | ACQUITY UPLC BEH Amide (1.7 µm, 130 Å, 2.1 x 150 mm, Waters Corporation, Milford, Massachusetts, USA) | | |
| **Temperature** | 50 °C | | |
| **Mobile Phase** | A: 95 % Water : 5 % Acetonitril (5 mM ammonium formate, pH 3.2) | | |
| | B: 95 % Acetonitril: 5 % Water (5 mM ammonium formate, pH 3.2) | | |

| | Time [min] | A [%] | B [%] |
|---|---|---|---|
| **Gradient** | 0 | 10 | 90 |
| | 5.33 | 12 | 88 |
| | 11.76 | 24 | 76 |
| | 15.00 | 24 | 76 |
| | 16.00 | 50 | 50 |
| | 20.00 | 50 | 50 |
| **Flow rate** | 0.45 mL/min | | |
| **Injection volume** | 5 µL | | |
| **Time** | 20 min | | |
| **Time of equilibration** | 10 min | | |
| **Auto-sampler temperature** | 20 °C | | |
| **Detection** | HDMS^{E} | | |
| | Mode: ESI-neg | | |
| | Capillary voltage: 3.0 kV | | |
| | Cone voltage: 40 V | | |
| | Source temperature: 120 °C | | |
| **MS parameter** | Drying gas: 450 °C, 580 L/h | | |
| | Cone gas: 50 L/h | | |
| | Scan area: 50 - 1600 m/z | | |
| | Low energy: 6 V | | |
| | High energy ramp: 20 - 40 V | | |
| **Sample concentration** | 100 µg/mL (80 % Acetonitrile in water) | | |

The isolated compounds were characterized due to the mass and fragment pattern. The corresponding parameters are shown in the following table:

| **Compound** | **Molecular formula** | **m/z precursor ion** | **m/z fragment ion** |
|---|---|---|---|
| **1** | C₃₈H₆₀O₁₈ | 803 [M-H] | 479 |
| **2** | C₃₈H₆₀O₁₈ | 803 [M-H] | 641 |
| **3** | C₄₄H₇₀O₂₃ | 965 [M-H] | 485 |
| **4** | C₄₄H₇₀O₂₃ | 965 [M-H] | 641 |
| **5** | C₄₄H₇₀O₂₃ | 965 [M-H] | 641 |
| **6** | C₄₄H₇₀O₂₃ | 965 [M-H] | 803 |
| **7** | C₅₀H₈₀O₂₈ | 1127 [M-H] | 647 |
| **8** | C₅₀H₈₀O₂₈ | 1127 [M-H] | 641 |
| **9** | C₅₀H₈₀O₂₈ | 1127 [M-H] | 641 |
| **10** | C₅₀H₈₀O₂₈ | 1127 [M-H] | 965 |
| **11** | C₅₀H₈₀O₂₈ | 1127 [M-H] | 803 |

### Example 4: Sensory evaluation

### Example 4.1: Sweetness enhancement, comparing rubusoside glucosides with the same number of glucose moieties

To determine a sweetness enhancement by fraction 2a (compound 3) fraction 2c (compounds 5 and 6) according to Example 2, three samples (1, 2and 3) of a 5 % sucrose solution were prepared. 80 ppm of fraction 2a were added to sample 2, 80 ppm of fraction 2c were added to sample 3 , whereas no further substance was added to sample 1. The samples were then tested in a tasting with a trained panel with regard to their sweetness enhancement.

Fraction 2c increased the sweetness of the sucrose solution with a probability of error of 10 % as shown in the below table:

| **Sample** | **Base** | **Addition** | **Panel (n)** | **Increase in sweetness** | **t-test** |
|---|---|---|---|---|---|
| 1 | 5 % sucrose | --- | 20 | --- | --- |
| 2 | 5 % sucrose | 80 ppm fraction 2a | 20 | 17.21 | 0.0819 |
| 3 | 5 % sucrose | 80 ppm fraction 2c | 20 | 30.37 | 0.0021 |

It was surprisingly found that fraction 2c provided a much stronger increase in sweetness than fraction 2a, even though the same amount of rubusoside glucosides was provided and all rubusoside glucosides tested contained 2 additional glucose moieties compared to rubusoside.

### Example 4.2: Comparison with other rubusoside glucosides

Consent tasting and determination of a sweet taste of the compounds was performed by a trained panel testing samples on a basis of a 5 % sucrose solution with 80 ppm of fraction 2a (compound 3), with 80 ppm of fraction 2c (compounds 5 and 6, wherein compound 5 represented approximately 66 % and compound 6 approximately 33 % of the compounds in fraction 2c), with 80 ppm of fraction 3c (compounds 9 and 10, wherein compound 9 represented approximately 66 % and compound 20 approximately 33 % of the compounds in fraction 2c) or with 80 ppm of fraction 3d (compound 11) according to Example 2 and comparing these samples with a 5 % sucrose solution. The evaluation was based on five descriptors (onset sweetness, overall sweetness, body, longlasting, off-notes). The results are depicted in Fig. 1.

| Fraction | Dosage | Onset Sweetness | Overall Sweetness | Body | Longlasting | Off-Notes |
|---|---|---|---|---|---|---|
| 5 % sucrose | | 4 | 5 | 5 | 0 | 0 |
| 2a | 80ppm | 5 | 6,5 | 6,5 | 1,5 | 1 |
| 2c | 80ppm | 7,5 | 8 | 8 | 3 | 1,5 |
| 3c | 80ppm | 5,5 | 7 | 6 | 2 | 1,5 |
| 3d | 80ppm | 6,5 | 8 | 7,5 | 2 | 1 |

It was surprisingly found that fraction 2c provided much higher scores in the evaluation of the descriptors onset sweetness, overall sweetness, body and longlasting than fraction 2a, even though the same amount of rubusoside glucosides was provided and the rubusoside glucosides tested all contain 2 additional glucose moieties compared to rubusoside.

Furthermore, it was surprisingly found that fraction 2c provided much higher or at least equal scores in the evaluation of the descriptors onset sweetness, overall sweetness, body and longlasting than fractions 3c or 3d. This was particularly surprising, since the same amount of rubusoside glucosides was provided and the rubusoside glucosides in fractions 3c and 3d each contain 3 additional glucose moieties compared to rubusoside, whereas the rubusoside glucosides in fraction 2c only contain 2 additional glucose moieties.

## Claims

1. Compound according to the following formula

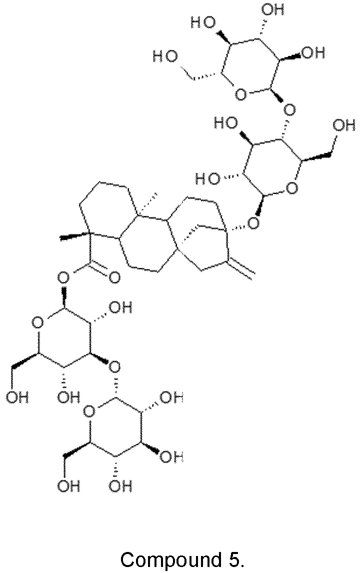

2. Mixture comprising the compound of claim 1.

3. Mixture according to claim 2, further comprising the compound according to the following formula

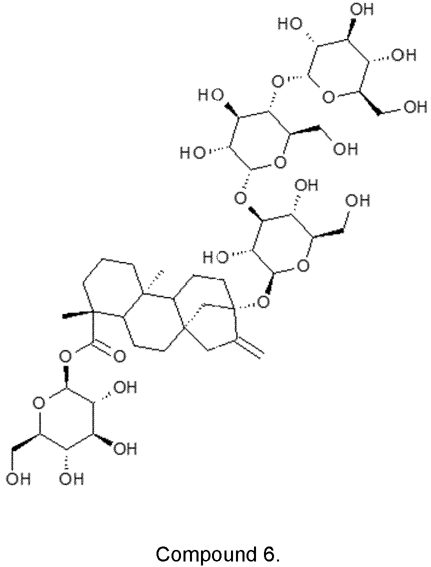

4. Mixture according to claims 2 or 3, wherein the mixture comprises
a) 40 to 90 wt.-% Compound 5 and
b) 10 to 60 wt.-% Compound 6,
based on the total weight of the mixture.

5. Mixture according to claims 3 or 4,
wherein the weight ratio of Compound 5 and Compound 6 is in a range of from 10:1 to 1:5, preferably in a range of from 7.5:1 to 1:4, preferably in a range of from 5:1 to 1:3, preferably in a range of from 3:1 to 1:2.

6. Method for producing a compound according claim 1 or a mixture according to any one of claims 2 to 5, comprising the following steps:
(i) Providing Rubusoside and providing a glucosyl donor, mixing the provided Rubusoside and the glucosyl donor,
(ii) dissolving the mixture of step (i) in a solvent, preferably water,
(iii) adding a glucanotransferase and reacting the dissolved mixture with the glucanotransferase to obtain glucosylated Rubusosides,
(iv) heating the mixture for inactivating the glucanotransferase,
(v) enriching the glucosylated Rubusosides in the mixture, preferably by column chromatography,
(vi) optionally: separating the enriched glucosylated Rubusosides of the mixture, preferably by liquid chromatography.

7. Method according to claim 6, wherein the method comprises step (vi) and step (vi) comprises a first liquid chromatography, in which the glucosylated Rubusosides are separated into different fractions according to their degree of glucosylation, and a second liquid chromatography, in which the obtained fractions are further separated into the specific isomers of the glucosylated Rubusosides.

8. Mixture according to any one of claims 2 to 5, wherein the mixture is obtainable or obtained by a method according to claim 6 or 7.

9. Flavour composition comprising the compound according to claim 1 or a mixture according to any one of claims 2 to 5 or 8, wherein the total weight of the compound according to claim 1 is at least 0.05 wt.-%, preferably at least 0.1 wt.-%, particularly preferably at least 0.25 wt.-%, based on the total weight of the flavour composition.

10. Flavour composition comprising the mixture according to any one of claims 3 to 5 or 8, comprising compound 6, as defined in claim 3,
wherein the total weight of the compound according to claim 1 and compound 6 is at least 0.05 wt.-%, preferably at least 0.1 wt.-%, particularly preferably at least 0.25 wt.-%, based on the total weight of the flavour composition.

11. Composition serving for food or pleasure or pharmaceutical composition comprising the compound according to claim 1 or a mixture according to any one of claims 2 to 5 or 8 or a flavour composition according to claims 9 or 10.

12. Use of the compound according to claim 1 or a mixture according to any one of claims 2 to 5 or 8 or a flavour composition according to claims 9 or 10 for imparting, modifying and/or enhancing a sweet taste impression.

13. Method for imparting, modifying and/or enhancing a sweet taste impression, comprising the following steps:
(i) Providing a compound according to claim 1 or a mixture according to any one of claims 2 to 5 or 8 or a flavour composition according to claims 9 or 10,
(ii) mixing the compound or mixture or flavour composition provided in step (i) with a substance or product, of which a sweet taste impression shall be imparted, modified and/or enhanced.

## Patentansprüche

1. Verbindung gemäß der nachfolgenden Formel

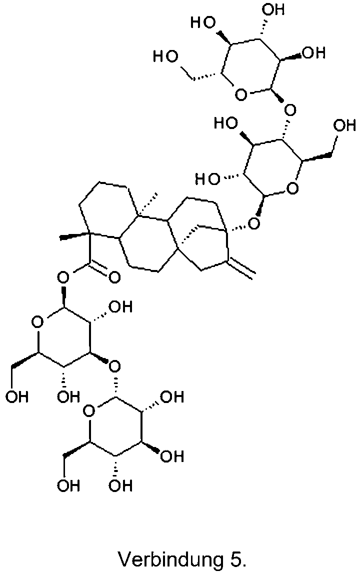

2. Mischung umfassend die Verbindung gemäß Anspruch 1.

3. Mischung gemäß Anspruch 2, weiter umfassend die Verbindung gemäß der nachfolgenden Formel

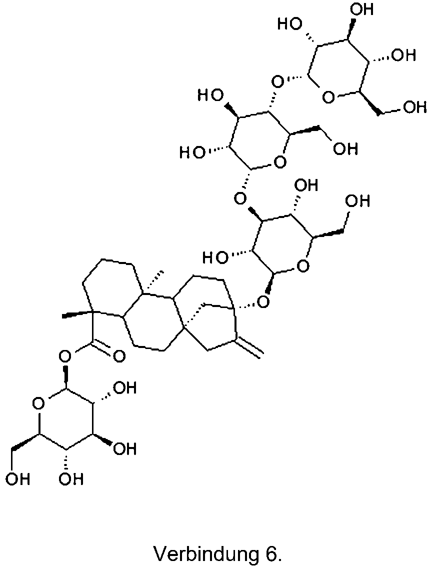

4. Mischung gemäß Anspruch 2 oder 3, wobei die Mischung umfasst
a) 40 bis 90 Gew.-% Verbindung 5 und
b) 10 bis 60 Gew.-% Verbindung 6,
bezogen auf das Gesamtgewicht der Mischung.

5. Mischung gemäß Anspruch 3 oder 4,
wobei das Gewichtsverhältnis von Verbindung 5 und Verbindung 6 in einem Bereich von 10:1 bis 1:5 liegt, vorzugsweise in einem Bereich von 7,5:1 bis 1:4, vorzugsweise in einem Bereich von 5:1 bis 1:3, vorzugsweise in einem Bereich von 3:1 bis 1:2.

6. Verfahren zum Herstellen einer Verbindung gemäß Anspruch 1 oder einer Mischung gemäß einem der Ansprüche 2 bis 5, umfassend die folgenden Schritte:
(i) Bereitstellen von Rubusosid und Bereitstellen eines Glukosyl-Donors, Mischen des bereitgestellten Rubusosids und des Glukosyl-Donors,
(ii) Lösen der Mischung aus Schritt (i) in einem Lösungsmittel, vorzugsweise Wasser,
(iii) Hinzugeben einer Glukanotransferase und Reagieren der gelösten Mischung mit der Glukanotransferase, um glukosylierte Rubusoside zu erhalten,
(iv) Erhitzen der Mischung, um die Glukanotransferase zu inaktivieren,
(v) Anreichern der glukosylierten Rubusoside in der Mischung, vorzugsweise durch Säulenchromatographie,
(vi) optional: Auftrennen der angereicherten glukosylierten Rubusoside der Mischung, vorzugsweise durch Flüssigkeitschromatographie.

7. Verfahren gemäß Anspruch 6, wobei das Verfahren Schritt (vi) umfasst und Schritt (vi) eine erste Flüssigkeitschromatographie umfasst, in der die glukosylierten Rubusoside in verschiedene Fraktionen aufgetrennt werden, abhängig von deren Glukosylierungsgrad, und eine zweite Flüssigkeitschromatographie, in der die erhaltenen Fraktionen weiter aufgetrennt werden in die spezifischen Isomere der glukosylierten Rubusoside.

8. Mischung gemäß einem der Ansprüche 2 bis 5, wobei die Mischung herstellbar oder hergestellt ist durch ein Verfahren gemäß Anspruch 6 oder 7.

9. Geschmacksstoffzusammensetzung umfassend die Verbindung gemäß Anspruch 1 oder eine Mischung gemäß einem der Ansprüche 2 bis 5 oder 8, wobei das Gesamtgewicht der Verbindung gemäß Anspruch 1 mindestens 0,05 Gew.-% beträgt, vorzugsweise mindestens 0,1 Gew.-%, besonders bevorzugt mindestens 0,25 Gew.-%, bezogen auf das Gesamtgewicht der Geschmacksstoffzusammensetzung.

10. Geschmacksstoffzusammensetzung umfassend die Mischung gemäß einem der Ansprüche 3 bis 5 oder 8, umfassend Verbindung 6, wie in Anspruch 3 definiert,
wobei das Gesamtgewicht der Verbindung gemäß Anspruch 1 und Verbindung 6 mindestens 0,05 Gew.-% beträgt, vorzugsweise mindestens 0,1 Gew.-%, besonders bevorzugt mindestens 0,25 Gew.-%, bezogen auf das Gesamtgewicht der Geschmacksstoffzusammensetzung.

11. Nahrungsmittel- oder Genusszusammensetzung oder pharmazeutische Zusammensetzung umfassend die Verbindung gemäß Anspruch 1 oder eine Mischung gemäß einem der Ansprüche 2 bis 5 oder 8 oder eine Geschmacksstoffzusammensetzung gemäß Anspruch 9 oder 10.

12. Verwendung der Verbindung gemäß Anspruch 1 oder einer Mischung gemäß einem der Ansprüche 2 bis 5 oder 8 oder einer Geschmacksstoffzusammensetzung gemäß Anspruch 9 oder 10 zum Hervorrufen, Modifizieren und/oder Verstärken eines süßen Geschmackseindrucks.

13. Verfahren zum Hervorrufen, Modifizieren und/oder Verstärken eines süßen Geschmackseindrucks umfassend die nachfolgenden Schritte:
(i) Bereitstellen einer Verbindung gemäß Anspruch 1 oder einer Mischung gemäß einem der Ansprüche 2 bis 5 oder 8 oder einer Geschmacksstoffzusammensetzung gemäß Anspruch 9 oder 10,
(ii) Mischen der in Schritt (i) bereitgestellten Verbindung oder Mischung oder Geschmacksstoffzusammensetzung mit einer Substanz oder einem Produkt, bei der / dem ein süßer Geschmackseindruck hervorgerufen, modifiziert und/oder verstärkt werden soll.

## Revendications

1. Composé selon la formule suivante:

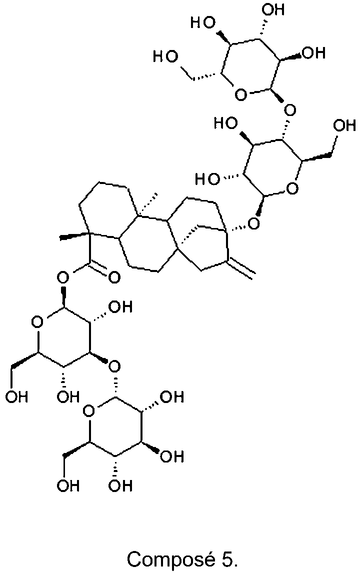

2. Mélange comprenant le composé selon la revendication 1.

3. Mélange selon la revendication 2, comprenant en outre le composé selon la formule suivante:

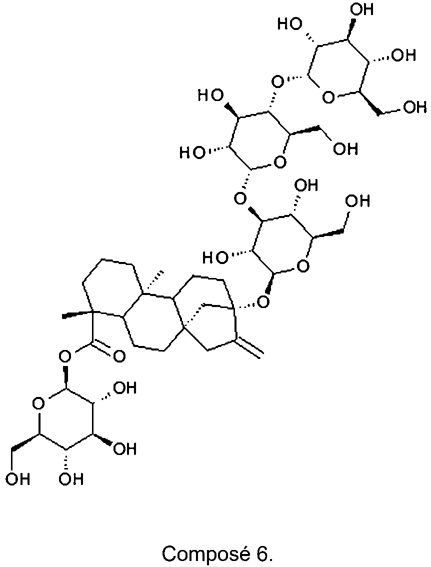

4. Mélange selon les revendications 2 ou 3, dans lequel le mélange comprend:
a) 40 à 90 % en poids du composé 5 et
b) 10 à 60 % en poids du composé 6,
par rapport au poids total du mélange.

5. Mélange selon les revendications 3 ou 4,
dans lequel le rapport pondéral du composé 5 et du composé 6 se situe dans une plage allant de 10 : 1 à 1 : 5, de préférence dans une plage allant de 7,5 : 1 à 1 : 4, de préférence dans une plage allant de 5 : 1 à 1 : 3, de préférence dans une plage allant de 3 : 1 à 1 : 2.

6. Procédé de production d'un composé selon la revendication 1 ou d'un mélange selon l'une quelconque des revendications 2 à 5, comprenant les étapes suivantes consistant à:
(i) fournir du rubusoside et fournir un donneur de glucosyl, puis mélanger le rubusoside et le donneur de glucosyl fournis,
(ii) dissoudre le mélange de l'étape (i) dans un solvant, de préférence de l'eau,
(iii) ajouter une glucanotransférase et faire réagir le mélange dissous avec la glucanotransférase pour obtenir des rubusosides glycosylés,
(iv) chauffer le mélange pour inactiver la glucanotransférase,
(v) enrichir les rubusosides glycosylés dans le mélange, de préférence par chromatographie sur colonne,
(vi) en option: séparer les rubusosides glycosylés enrichis du mélange, de préférence par chromatographie liquide.

7. Procédé selon la revendication 6, dans lequel le procédé comprend l'étape (vi), et l'étape (vi) comprend une première chromatographie liquide au cours de laquelle les rubusosides glycosylés sont séparés en différentes fractions selon leur degré de glucosylation, et une deuxième chromatographie liquide au cours de laquelle les fractions obtenues sont encore séparées en isomères spécifiques des rubusosides glycosylés.

8. Mélange selon l'une quelconque des revendications 2 à 5, dans lequel le mélange peut être obtenu ou est obtenu par un procédé selon la revendication 6 ou 7.

9. Composition d'aromatisant comprenant le composé selon la revendication 1 ou un mélange selon l'une quelconque des revendications 2 à 5 ou 8, dans laquelle le poids total du composé selon la revendication 1 est d'au moins 0,05 % en poids, de préférence d'au moins 0,1 % en poids, de manière particulièrement préférée d'au moins 0,25 % en poids, par rapport au poids total de la composition d'aromatisant.

10. Composition d'aromatisant comprenant le mélange selon l'une quelconque des revendications 3 à 5 ou 8, comprenant le composé 6, tel que défini dans la revendication 3,
dans laquelle le poids total du composé selon la revendication 1 et du composé 6 est d'au moins 0,05 % en poids, de préférence d'au moins 0,1 % en poids, de manière particulièrement préférée d'au moins 0,25 % en poids, par rapport au poids total de la composition d'aromatisant.

11. Composition destinée à l'alimentation ou au plaisir ou composition pharmaceutique, comprenant le composé selon la revendication 1 ou un mélange selon l'une quelconque des revendications 2 à 5 ou 8, ou une composition d'aromatisant selon les revendications 9 ou 10.

12. Utilisation du composé selon la revendication 1 ou d'un mélange selon l'une quelconque des revendications 2 à 5 ou 8, ou d'une composition d'aromatisant selon les revendications 9 ou 10, pour conférer, modifier et/ou intensifier une impression gustative sucrée.

13. Procédé pour conférer, modifier et/ou intensifier une impression de goût sucré, comprenant les étapes suivantes consistant à:
(i) fournir un composé selon la revendication 1 ou un mélange selon l'une quelconque des revendications 2 à 5 ou 8 ou une composition d'aromatisant selon les revendications 9 ou 10,
(ii) mélanger le composé ou le mélange ou la composition d'aromatisant fourni(e) à l'étape (i), avec une substance ou un produit à laquelle/auquel (dans laquelle/lequel) une impression de goût sucré doit être conférée, modifiée et/ou intensifiée.
